# EUROPEAN PATENT SPECIFICATION

(11) **EP 4 039 295 B1**
(45) Date of publication and mention of the grant of the patent: **12.03.2025**
(21) Application number: 22165184.7
(22) Date of filing: 24.04.2018
(51) Int. Cl.: A61M 5/31, A61M 5/315

(54) **MEDICAL DELIVERY DEVICES WITH INHIBITED OXYGEN PERMEATION**
MEDIZINISCHE FREISETZUNGSVORRICHTUNG MIT INHIBIERTER SAUERSTOFFPERMEATION
DISPOSITIFS D'ADMINISTRATION MÉDICALE À PERMÉATION D'OXYGÈNE INHIBÉE

(43) Date of publication of application: 10.08.2022
(62) Divisional of application: 18723307.7
(73) Proprietor: W. L. Gore & Associates, Inc., Newark, DE 19711 (US)
(72) Inventor: CULLY, Edward, H., Flagstaff, 86004 (US); GUNZEL, Edward, C., Oxford, 19363 (US); SCOTTI, Christine, M., Newark, 19711 (US)
(74) Representative: HGF

(56) References cited:
- WO-A1-2014/190225
- WO-A2-03/039632

## Description

### TECHNICAL FIELD

The present disclosure relates to medical delivery devices, and in particular, to syringes, auto-injectors, and pens configured to inhibit oxygen permeation into fluids or substances contained within the medical delivery device.

### BACKGROUND

Medical delivery devices such as syringes, auto-injectors, and pens typically include a barrel, a stopper positioned within the barrel, and a plunger rod or actuation mechanism to displace the stopper. As the pharmaceutical industry seeks new and more convenient drug delivery methods, pre-filled syringes, auto-injectors, and pens have emerged as the preferred choice for unit dose medication delivery. Pre-filled syringes minimize drug waste and increase product life span, while enabling patients to self-administer injectable drugs. In particular, pre-filled syringes, auto-injectors, and pens eliminate dosing errors and prevent overfilling. Drug instability over an extended period of time is one problem that persists with pre-filled syringes. Drug instability may result, for example, from interactions between the syringe and the fluid compositions contained therein.

Thus, the design and quality of pre-filled syringes help to ensure that the fluid composition contained therein remains uncontaminated. Contamination may result, for example, from contact between the fluid composition and any of several different components of the syringe, auto-injectors, and pens. For example, a syringe auto-injectors, and pens may include a barrel with a stopper slidably fitted within the barrel and may have a plunger rod for actuation of the syringe and delivery of a medicament. The stopper is generally constructed of an elastomer. Conventionally, silicone oil is applied to the stopper and/or the barrel to reduce sliding friction between the stopper and barrel and to improve the seal therebetween. The silicone oil allows the stopper to slide easily within the barrel when administering a dose, which may ensure the full dosage is administered.

The use of silicone in a pre-filled syringe, auto-injector, or pen is a concern in the industry because the oil may degrade the medicament and/or because an amount of silicone may be injected with it. The use of silicone in a pre-filled syringe, auto-injectors, and pens may be of particular concern with regard to biopharmaceuticals because it may cause aggregation of certain proteins.

In addition, some fluid compositions in pre-filled syringes auto-injectors, and pens are oxygen-sensitive and may oxidize or otherwise be adversely impacted by coming into contact with oxygen. Such oxygen sensitivity can present an issue for pre-filled syringes, auto-injectors, and pens, which typically permit a small amount of air to infiltrate into the syringe during storage. As a result, oxygen sensitive drugs or drug formulations may require additional excipients, packaging, and/or manufacturing steps for stability enhancement and degradation prevention. These approaches, however, add complexity to the overall construction of the device or drug formulation.

Patent application document WO 03/039632 A2 discloses a syringe barrel which may be formed from a rubber or other material that functions as an oxygen absorbing material or an oxygen barrier material.

Thus, there exists a need for silicone free pre-filled syringes, auto-injectors, and pens that are configured to inhibit or prevent oxygen permeation into the barrel of the syringe, auto-injector, or pen.

### SUMMARY OF THE INVENTION

The present disclosure is directed to medical delivery devices (e.g., syringes, auto-injectors, and pens) that include a silicone free barrel having proximal and distal ends and an interior surface that defines a fluid chamber for the containment of at least one therapeutic, an elastomeric stopper that has an outer surface that forms a fluid-tight seal with the interior surface of the barrel, and a plunger rod or actuation mechanism to displace the stopper within the barrel. The barrel has a region of reduced diameter located at the proximal end of the barrel to enhance an interference fit between the elastomeric stopper and the interior surface of the barrel. The stopper includes at least one rib in abutting contact with the region of reduced diameter while the stopper is in an undepressed state. The region of reduced diameter has a diameter that is less than the diameter of the remainder of the barrel. The barrel may include a transition section (e.g. a frusticonicial or tapered transition section) positioned between the region of reduced diameter at the proximal end and the region of greater diameter at the distal end to provide a gradual transition therebetween.

A coating material is provided on a portion of the interior surface of the barrel to form the region of reduced diameter. At least one rib of the stopper is in contact with the coating material in an undepressed state. In some embodiments, the coating material may include an oxygen-absorbing material, and oxygen-adsorbing material, or a combination of the two. In another embodiment, the coating includes materials that are low in oxygen permeability. In yet another embodiment, the coating is formed of a material that is conformable and has a low coefficient of friction to enable an improved seal at the proximal end of the medical device while also enabling reduced break and slide forces. In exemplary embodiments, the coating material does not extend more than half the length of the barrel.

In another embodiment, at least one insert is in contact with the interior surface of the barrel to form the region of reduced diameter. The insert is formed of or includes a gas barrier to inhibit oxygen infiltration into the barrel. The insert may be affixed to the interior surface of the barrel (e.g. via an adhesive) or it may be integrally formed on the interior surface of the barrel. In one embodiment, the insert is a solid polymer tube that is bonded to the interior surface of the barrel.

In a further embodiment, the region of reduced diameter includes a laminate having one or more fluoropolymer membranes attached to the interior surface of the barrel. The laminate may be formed of a single high strength, thin film (e.g., PTFE) or multiple high strength, thin films. The thin films may have a thicknesses ranging from about 0.5 µm to about 100 µm. In some embodiments, the laminate may be bonded or otherwise attached to the interior surface of the barrel. In an alternate embodiment, the laminate can be thermoformed on the interior surface of the barrel.

In yet another embodiment, the present disclosure relates to a medical device (e.g., syringes, auto-injectors, and pens) that includes a silicone free barrel having proximal and distal ends and an interior surface defining a fluid chamber for containment of at least one therapeutic, and an elastomeric stopper having an outer surface that forms a fluid-tight seal with the interior surface of the barrel, and a plunger rod or actuation mechanism to displace the stopper within the barrel. The stopper includes a plurality of ribs and a valley between each pair of ribs. At least one ring member is provided within one or more valleys of the stopper. The ring member may include an oxygen-absorbing material and/or an oxygen-adsorbing material that binds to and/or otherwise removes any oxygen that may permeate into the valleys. In some embodiments, each valley of the elastomeric stopper includes at least one ring member. The medical device may further include a coating on a portion of the interior surface of the barrel adjacent to the proximal end of the barrel to form a coating region that forms a region of reduced diameter which has a diameter that is less than the diameter of the uncoated region of the barrel.

In a further embodiment, the present disclosure relates to a medical device (e.g., syringes, auto-injectors, and pens) that includes a silicone free barrel having proximal and distal ends and an interior surface defining a fluid chamber for the containment of at least one therapeutic, an elastomeric stopper having an outer surface that forms a fluid-tight seal with the interior surface, a plunger rod attached to the stopper and mounted within the syringe barrel for moving the stopper distally within the barrel, and a barrier coating on a portion of the stopper. The barrier coating may include an oxygen barrier material. The barrier coating may surround the portion of the stopper that is not exposed to the fluid chamber. In some embodiments, the barrier coating surrounds the entirety of the stopper as long as the barrier coating is inert to the therapeutic contained within the barrel of the syringe. The stopper may include an elastomeric body having a plurality of ribs and a valley between each pair of ribs. The coating may be provided on the ribs and in the valley between each pair of ribs. In some aspects, the plunger rod further includes the coating.

These and other embodiments, along with many of their advantages and features, are described in more detail in conjunction with the below description and attached non-limiting figures.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1a shows a cross-sectional view of a syringe barrel having a region of reduced diameter according to some embodiments.
FIG. 1b shows an exploded view of a syringe barrel having a region of reduced diameter according to some embodiments;
FIGS. 2a-c show process steps for coating an interior surface of a syringe barrel in accordance with some embodiments;
FIG. 3 shows a cross-sectional view of a syringe barrel with a coating on the interior surface thereof according to some embodiments ;
FIG. 4 shows an exploded view of a stopper rib contacting the interior surface of the syringe barrel of FIG. 3 according to some embodiments;
FIG. 5 shows a cross-sectional view of the stopper depressed into the syringe barrel according to some embodiments ;
FIG. 6 shows a perspective view of a syringe with ring members around the stopper valleys according to some embodiments;
FIG. 7 shows a cross-sectional view of the syringe of FIG. 6 according to some embodiments;
FIG. 8 shows a cross-sectional view of a syringe with a coated stopper according to some embodiments ;
FIG. 9 shows a cross-sectional view of a syringe with a coated stopper and plunger rod according to some embodiments;
FIGS. 10-17 are schematic illustrations of cross-sectional side views of stoppers depicting varying types of laminates applied thereto according to some embodiments; and
FIG. 18 is a schematic cross section of an auto-injector according to some embodiments.

### DETAILED DESCRIPTION

Persons skilled in the art will readily appreciate that various aspects of the present disclosure can be realized by any number of methods and apparatus configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting. The terms "silicone" and "silicone oil" may be used interchangeably herein.

The present disclosure relates to medical delivery devices (e.g. syringes, auto-injectors, and pens) that are free or substantially free of silicone that are used for storing and delivering a fluid for medical use. As used herein, the term "substantially free" is meant to denote that the barrel of the syringe and/or the stopper has an unquantifiable or trace amount of silicone or other liquid lubricants. The medical delivery devices are configured to inhibit or prevent oxygen permeation into the barrel of the medical delivery device. The present disclosure provides a stopper that is suitable for use in syringes free or substantially free of silicone or other liquid lubricants which inhibits or prevents the ingress of oxygen into the barrel and in contact with the therapeutic liquid in the medical delivery device. For ease of description, the disclosure hereafter is directed to a syringe, although the description is equally applicable to an auto-injector or pen. In addition, in alternate embodiments, the barrel may include silicone (or another lubricant) and such an embodiment is considered to be within the purview of this disclosure.

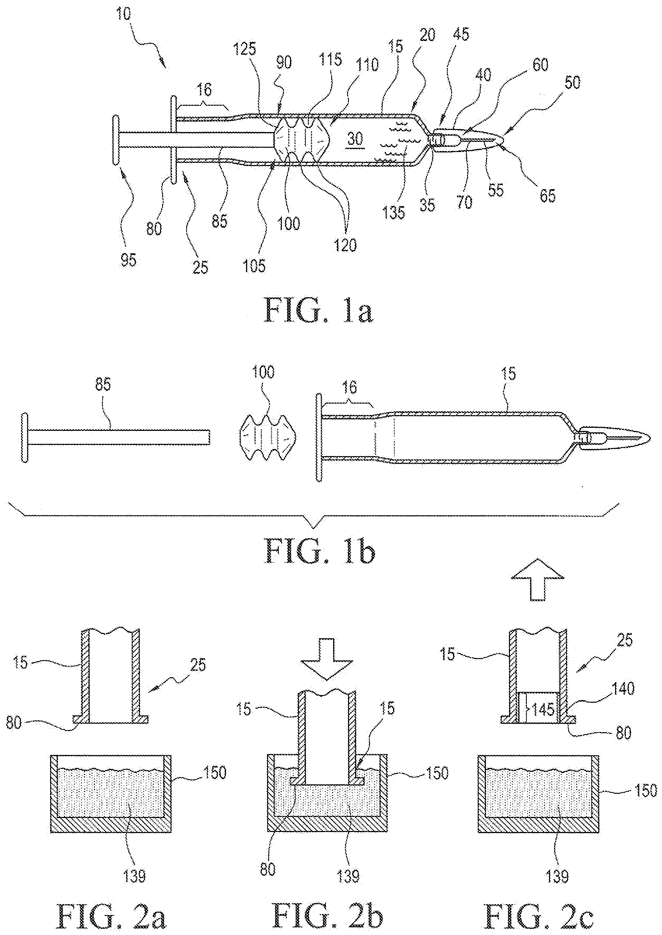

FIG. 1a shows a syringe 10 (e.g., a prefilled syringe) in accordance with one or more embodiments. The syringe 10 includes a barrel 15 that is free or substantially free of silicone and which has opposed distal and proximal ends 20 and 25, respectively, and a fluid chamber 30 positioned between distal and proximal ends 20 and 25. In certain embodiments, the barrel 15 has a hydrophilic interior surface. Distal end 20 of barrel 15 may include an injection port 35 extending therethrough and communicating with the fluid chamber 30. In some embodiments, the barrel is formed of a glass material, such as, for example a borosilicate glass, that is free of silicone or other lubricants.

In some embodiments, a cap 40 is disposed at the distal end 20 of barrel 15. The cap 40 includes a proximal end 45 mated to the distal end 20 or injection port 35 and a closed distal end 50. The cap 40 inhibits or prevents ambient air from communicating with the fluid chamber 30 through the distal end 20 or injection port 35. Optionally, a piercing element 55 is also disposed at the distal end 20 of the barrel 15. The piercing element 55, e.g., needle, includes a distal end 65 and a proximal end 60 which is mated to the distal end 20 or injection port 35. As should be understood to one skilled in the art, it is within the purview of the present disclosure that in some embodiments, the injection port 35 can include a sharply pointed needle cannulae, or blunt-ended cannulae, such as those employed with so-called needleless systems. For purposes of illustration but not of limitation, as herein shown, the piercing element 55 is formed as a sharply pointed, elongated needle cannula including the proximal end 60, a sharply pointed distal end 65 and a lumen 70 extending between the proximal end 60 and the distal end 65. Proximal end 60 of the needle cannula may be rigidly mounted to the injection port 35 of the barrel 15.

In some embodiments, a cap 40 is mounted over the injection port 35 and is releasably engaged to the injection port 35 of the barrel 15. The cap 40, which may be formed from a rigid material such as plastic, or which can be formed from a flexible material such as rubber, or from like materials or combinations known to the skilled artisan, may be configured with appropriately dimensioned sealing elements, or the like. Thus, cap 40 inhibits or prevents ambient air from communicating with the fluid chamber 30 through injection port 35.

The proximal end 25 of barrel 15 may include flanges 80 used as a finger holder for pressing and pulling a plunger rod 85 within barrel 15. The plunger rod 85 has opposed distal and proximal ends 90 and 95 with a stopper 100 attached at the distal end 90. The stopper 100 includes opposed proximal and distal ends 105 and 110. As the skilled artisan will appreciate, the stopper 100 may include one or more circumferentially extending annular ribs that form a fluid-tight seal with the barrel 15. In at least one embodiment, the stopper 100 includes two or more ribs 120. In embodiments having a plurality of ribs, the stopper includes a valley 115 between each pair of adjacent ribs 120.

The barrel may be formed of a substantially rigid or hard material, such as a glass material (e.g., borosilicate glass), a ceramic material, one or more polymeric materials (e.g., polypropylene, polyethylene, and copolymers thereof), a metallic material, or a plastic material (e.g., cyclic olefin polymers (COC) and cyclic olefin copolymers (COP), and combinations thereof. In certain embodiments, the barrel is formed of glass (e.g., bare glass, without any lubricants thereon), resin, plastic, metal, or like materials and has an interior wall characterized by the absence of a lubricant such as, but not limited to, silicone or silicone oil.

The stopper 100 may be formed of an elastomeric body 125. The elastomeric body 125 can include any elastomer suitable for the application, most notably rubbers constructed from butyl, bromobutyl, chlorobutyl, silicone, nitrile, styrene butadiene, polychloroprene, ethylene propylene diene, fluorelastomers, or combinations or blends of any of the foregoing. The materials of the elastomeric body 125 are chosen to provide low coefficient of friction, compliance, low extractables and leachables, good barrier properties as they relate to extractables and leachables from the elastomeric body 125, and good air and liquid impermeability.

In some embodiments, the stopper 100 may further include a low friction barrier film surrounding the elastomeric body 125. The barrier film may inhibit materials from leaching from the elastomer material or from extraction of compounds from medicants by the elastomer. The materials of the barrier film are chosen to provide low coefficient of friction, compliance, low extractables and leachables, good barrier properties as they relate to extractables and leachables from the elastomer body 125. For example, the barrier film may include one or more fluoropolymer films such as, but not limited to, polytetrafluoroethylene (PTFE) or expanded polytetrafluoroethylene (ePTFE) films.

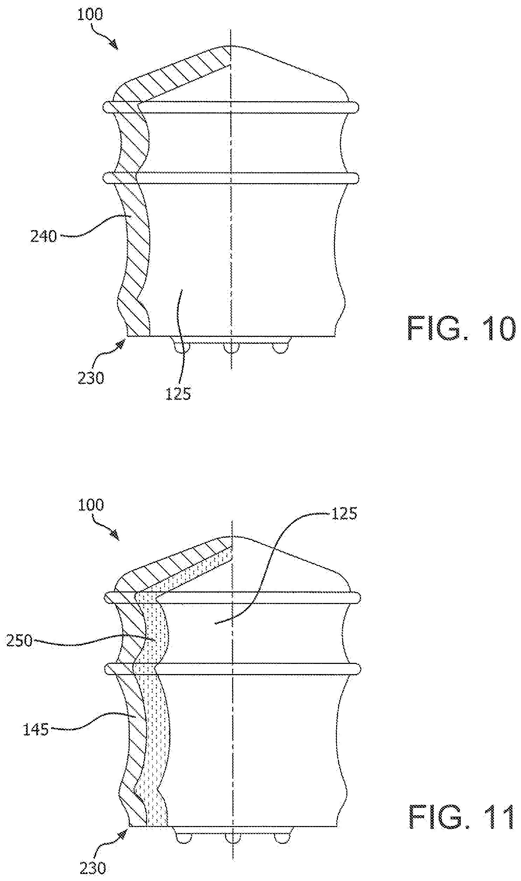

In some embodiments, the stopper 100 may be formed of an elastomeric body 125 and one or more laminate layers 230. The laminate layers 230 may include a single layer of a polymer barrier layer 240. FIG. 10 depicts such a stopper 100 that includes an elastomeric body 125 and a single layer comprising a barrier layer 240.

Examples of elastomers that can be used to form the elastomeric body 125 include any elastomer suitable for the application, most notably rubbers constructed from butyl, bromobutyl, chlorobutyl, silicone, nitrile, styrene butadiene, polychloroprene, ethylene propylene diene, fluoroelastomers, thermoplastic elastomers (TPE), thermoplastic vulcanizates (TPV), materials sold under the trade name VITON^{®}, and combinations and blends thereof. Exemplary elastomeric materials include, but are not limited to, butyl rubber, bromobutyl rubber, chlorobutyl rubber, silicone, nitrile, styrene butadiene, polychloroprene, ethylene propylene diene, fluoroelastomers and combinations thereof.

Examples of fluoropolymers that can be used to form the barrier layer 240 include any fluoropolymer suitable for the application, most notably a densified expanded fluoropolymer such as densified expanded polytetrafluoroethylene (ePTFE). Other suitable fluoropolymers include, but are not limited to, expanded polytetrafluoroethylene (ePTFE), fluorinated ethylene propylene (FEP), polyvinylidene fluoride, polyvinylfluoride, perfluoropropylvinylether, perfluoroalkoxy polymers, tetrafluoroethylene (TFE), Parylene AF-4, Parylene VT-4, and copolymers and combinations thereof. Non-fluoropolymers such as, but not limited to, polyethylene, polypropylene, Parylene C, and Parylene N may be used to form the barrier layer 240.

In one or more embodiment, the barrier layer 240 may include, or be formed of, one or more of the following materials: ultra-high molecular weight polyethylene as taught in U.S. Patent No. 9,732,184 to Sbriglia; polyparaxylylene as taught in U.S. Patent Publication No. 2016/0032069 to Sbriglia; polylactic acid as taught in U.S. Patent No. 9,732,184 to Sbriglia, et al.; and/or VDF-co-(TFE or TrFE) polymers as taught in U.S. Patent No. 9,441,088 to Sbriglia.

In some embodiments, the barrier layer 240 may include, or be formed of, an expanded fluoropolymer or a densified expanded fluoropolymer, preferably an expanded polytetrafluoroethylene (ePTFE) or a densified expanded polytetrafluoroethylene. A densified ePTFE film may be prepared in the manner described in U.S. Patent No. 7,521,010 to Kennedy, et al.*,* U.S. Patent 6,030,694 to Dolan et al., U.S. Patent 5,792,525 to Fuhr et al., or U.S. Patent 5,374,473 to Knox et al. Expanded copolymers of PTFE, such as are described in U.S. Patent No. 5,708,044 to Branca, U.S. Patent No. 6,541,589 to Baillie, U.S. Patent No. 7,531,611 to Sabol et al., U.S. Patent No. 8,637,144 to Ford, and U.S. Patent No. 9,139,669 to Xu et al. may be utilized, particularly if they are densified.

The barrier layer 240 may also include an expanded polymeric material including a functional tetrafluoroethylene (TFE) copolymer material having a microstructure characterized by nodes interconnected by fibrils, where the functional TFE copolymer material includes a functional copolymer of TFE and PSVE (perfluorosulfonyl vinyl ether), or TFE with another suitable functional monomer, such as, but not limited to, vinylidene fluoride (VDF), vinyl acetate, or vinyl alcohol. The functional TFE copolymer material may be prepared, for example, according to the methods described in U.S. Patent No. 9,139,669 to Xu et al. *or* U.S. Patent No. 8,658,707 to Xu et al.

In another embodiment, as shown in FIG. 11, the laminate layers 230 may include a composite material that includes a barrier layer 245 and a porous layer 250. The barrier layer 245 can include a fluoropolymer such as a densified expanded fluoropolymer (*e.g.*, densified expanded polytetrafluoroethylene), polytetrafluoroethylene (PTFE), expanded polytetrafluoroethylene (ePTFE), fluorinated ethylene propylene (FEP), polyvinylidene fluoride, polyvinylfluoride, perfluoropropylvinylether, perfluoroalkoxy polymers, tetrafluoroethylene (TFE), Parylene AF-4, Parylene VT-4, and copolymers and combinations thereof. Non-fluoropolymers such as polyethylene, polypropylene, Parylene C, and Parylene N may also be utilized in the barrier layer 245. It is to be appreciated that any of the materials set forth above with respect to the barrier layer 240 may be used in or as the barrier layer 245. The porous layer 250 may include ePTFE (for example, ePTFE as taught in U.S. Patent No. 6,541,589 to Baille) or other porous expanded (and often fibrilizing) fluoropolymers. The laminate layers 230 having the barrier layer 245 and the porous layer 250 may be constructed by coating or otherwise depositing the barrier polymer (*e.g*. fluoropolymer) onto the porous layer 250 to create the composite material. One such example of this is to deposit a granular or powdered fluoropolymer such as powdered PTFE onto the surface of a porous ePTFE layer in a coating process. The ePTFE layer should be constructed to be sufficiently thermally stable to allow heat treatment of the deposited granular or powdered fluoropolymer for the creation of a barrier layer or for the bonding of the deposited barrier layer to the porous ePTFE layer. It is to be noted that the porous (*e.g*., ePTFE) layer may be filled with an organic or inorganic material to provide color, lubricity, or other functional attributes.

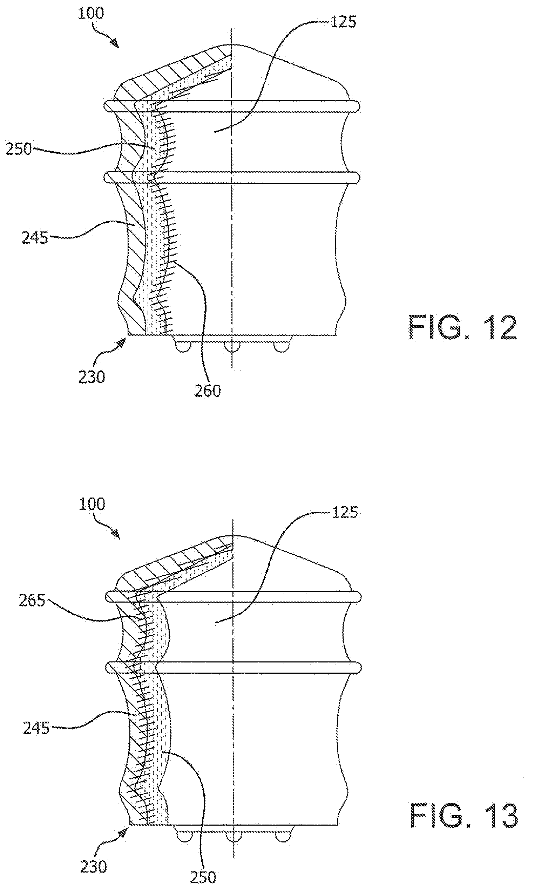

In accordance with some embodiments, the elastomer material of the elastomeric body 125 may at least partially penetrate the porous layer 250. FIG. 12 illustrates a cross-section of a stopper depicting the barrier layer 145, the porous layer 250, and the elastomeric body 125. Specifically, FIG. 12 shows a region of partial penetration 260 of the elastomer material of the elastomeric body 125 into the porous layer 250. Penetration of the elastomer material of the elastomeric body 125 into the porous layer 250 improves the bond between the elastomeric body 125 and the laminate layers 230.

In accordance with other aspects, the material of the barrier layer 245 may at least partially penetrate the porous layer 250. FIG. 13 illustrates a cross-section of a stopper depicting the barrier layer 245, the porous layer 250, and the elastomeric body 125. Specifically, FIG. 13 shows a region of partial penetration 265 of the material of the barrier layer 245 into the porous layer 250. Penetration of the material of the barrier layer 245 into the porous layer 250 improves the bond between the barrier layer 245 and the porous layer 250. The region of partial penetration 265 may also provide support for the barrier layer 245 to impart strength, toughness, compliance and stability, which may be beneficial in both the forming process and in the application.

In some embodiments, the barrier layer 245 may substantially fill the porous layer 250. In another aspect, the porous layer 250 may be filled to a substantially similar degree with barrier layer 245 and elastomer, leaving few open pores in the porous structure. In still another embodiment, both the barrier layer 245 and the elastomer partially fill the porous layer 250, while leaving some open pores between them. Other variations of penetration of elastomer and/or the barrier layer 245 may be readily apparent to one of skill in the art. Each may have advantages according to the specific application, with due consideration to the various desirable characteristics of the finished device, such as reduced friction, improved barrier properties, and improved sealing. The degree of penetration of either barrier polymer or elastomer may be controlled by any means known, but include variations in time, temperature, pressure, and porosity of the porous material. In one aspect the porous material may, for example have a porosity that varies with depth.

In yet another embodiment, the barrier layer 245 may be formed of a composite fluoropolymer or non-fluoropolymer material having a barrier layer and a tie layer such as is described in U.S. Patent Publication No. 2016/0022918 to Gunzel. It is to be noted that, as used herein, the term "tie layer" may include fluoropolymer and/or non-fluoropolymer materials. The tie layer can include, or be formed of, ePTFE or other porous expanded fluoropolymers (for example, ePTFE as taught in US 6,541,589 to Baille). Alternatively, the tie layer may be formed of, or include, non-fluoropolymer materials. Non-limiting examples of suitable non-fluoropolymer materials for use in or as the tie layer include non-fluoropolymer membranes, non-fluoropolymer microporous membranes, non-woven materials (*e.g*., spunbonded, melt blown fibrous materials, electrospun nanofibers), polyvinylidene difluoride (PVDF), nanofibers, polysulfones, polyethersulfones, polyarlysolfones, polyether ether ketone (PEEK), polyethylenes, polypropylenes, and polyimides.

In a further embodiment, the barrier layer 245 can be made by forming a thin densified composite comprising a porous ePTFE layer and a thermoplastic barrier layer. In this aspect, a thermoplastic having a surface with a low coefficient of friction is preferred. Accordingly, fluoropolymer based thermoplastics such as FEP, PFA, THV may be applicable. A barrier according to this aspect may be an FEP/ePTFE laminate obtained by following the process taught in WO 94/13469 to Bacino. The barrier may be formed at process temperatures above the softening temperature or even above the melt of the FEP film in a female cavity mold.

A composite material formed of an expanded fluoropolymer (*e.g.*, ePTFE) and a fluoropolymer based thermoplastic (*e.g*., FEP) described herein permits the formation of surprisingly thin, strong barrier films. In one embodiment, the ePTFE layer may act as a support during shape forming to allow thin barrier films. The porous ePTFE layer may also act as a reinforcement to the thermoplastic layer to maintain film strength and integrity of the barrier layer as described above, the ePTFE porous layer can also serve as a bonding layer when a portion of the ePTFE is allowed to remain porous and oriented toward the inside of the mold.

In another embodiment, the barrier layer 245 may comprise a composite of a densified ePTFE film and a thin layer of porous ePTFE bonded to the barrier layer film. The densified ePTFE film may be obtained as described in U.S. Patent No. 7,521,010 to Kennedy et al. The ePTFE / densified ePTFE composite may be combined in the manner described in U.S. Patent No. 6,030,694 to Dolan, et al. In this embodiment, the composite material comprises a layer of densified ePTFE film and a porous ePTFE layer. The porous ePTFE layer is constructed in a manner that it retains most of its porosity through thermoforming. It is also sufficiently compliant that it improves sealability against the syringe barrel wall. To accomplish this, at least a portion of the porous layer may remain sufficiently open after thermoforming and post compression molding with the elastomer. This open porosity allows some compressibility which may aid in the conformability and seal of the stopper to the surface.

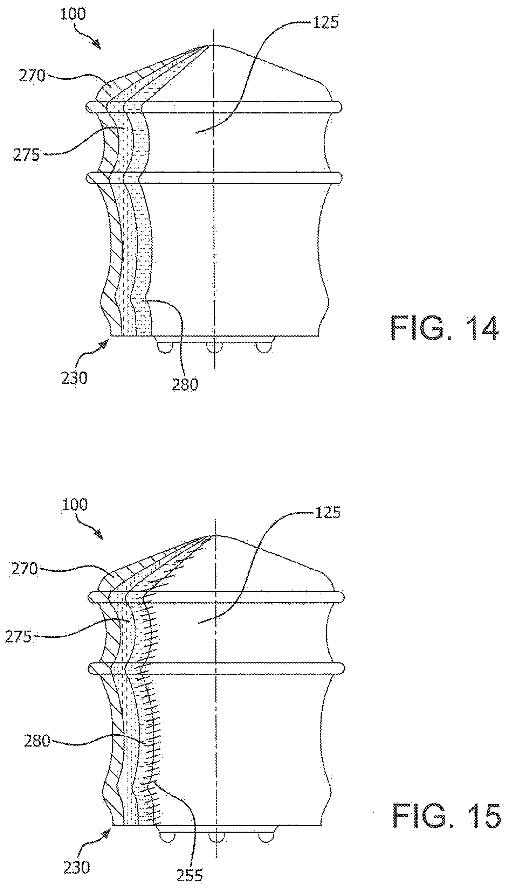

In another embodiment, as shown in FIG. 14, the laminate layer 230 includes a composite material having at least three layers, namely, a densified expanded fluoropolymer layer 270, a barrier melt fluoropolymer layer 275, and a porous layer 280. The densified expanded fluoropolymer layer 270 can may include or be formed of a densified ePTFE. The barrier melt fluoropolymer layer 275 may include a fluoropolymer such as a densified expanded fluoropolymer, PTFE, ePTFE, densified ePTFE, fluorinated ethylene propylene (FEP), polyvinylidene fluoride, polyvinylfluoride, perfluoropropylvinylether, perfluoroalkoxy polymers, and copolymers and combinations thereof. Non-limiting examples of non-fluoropolymers that may be utilized in the barrier melt layer 275 include polyethylene and polypropylene. The porous layer 280 may include or be formed of ePTFE or other porous expanded fluoropolymers. The laminate layers 230 having the densified expanded fluoropolymer layer 270, the barrier melt fluoropolymer layer 275 and the porous layer 280 may be constructed by coating or otherwise depositing the densified expanded fluoropolymer onto the porous layer to create the composite material. The densified ePTFE film, fluoropolymer, and porous layer may be thermoformed to make a preform, which may then be combined with the elastomeric body 125 by injection molding, compression molding, priming and post laminating around an elastomer perform, or other suitable methods known to the skilled artisan. In one non-limiting embodiment, the laminate layer 230 is formed of a densified fluoropolymer (*e.g.*, densified ePTFE), a thermoplastic adhesive (*e.g.,* FEP), and a porous fluoropolymer (*e.g.*, ePTFE).

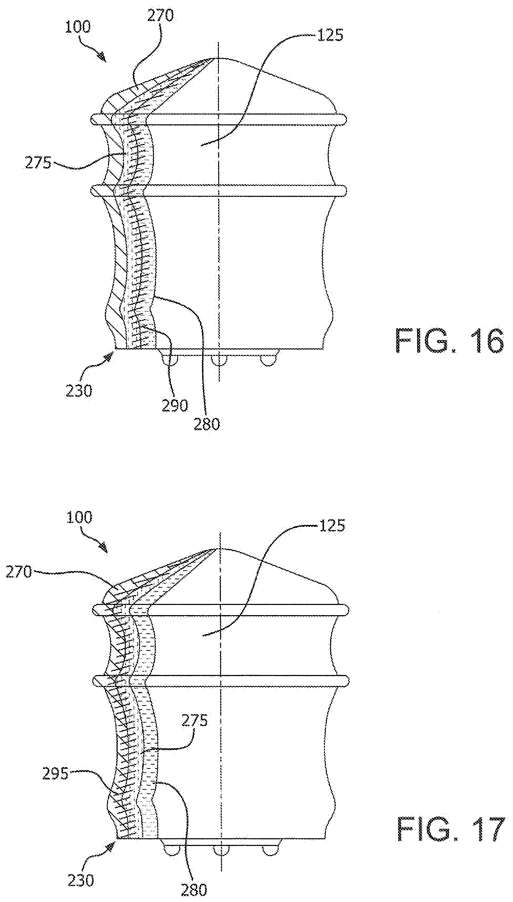

In accordance with some aspects, the elastomer material of the elastomeric body 125 may at least partially penetrate the porous layer 280 as shown in FIG. 15. In accordance with other embodiment, the material of the barrier melt fluoropolymer layer 275 may at least partially penetrate the porous layer 180 as shown in FIG. 16. In other embodiments, the material of the barrier melt fluoropolymer layer 275 may at least partially penetrate the densified expanded fluoropolymer layer 270, such as is depicted in FIG. 17.

It is to be appreciated that the stopper 100 may include various degrees of penetration of either the elastomer material or the barrier polymer into the porous material or the densified expanded fluoropolymer layer as depicted herein and as described in U.S. Patent No. 8,722,178 to Ashmead, et al.*,* U.S. Patent Publication No. 2012/0251748 to Ashmead, et al.*,* and U.S. Patent Publication No. 2016/0022918 to Ashmead, et al.*.* It is also to be appreciated that there are many variations of the processes described herein that could be utilized for forming the stopper 100 without departing from the scope of the invention as defined in the appended claims.

In one embodiment, the elastomeric body 125 of the stopper may have thereon an ePTFE film or densified ePTFE film. The high strength expanded film allows for forming extremely thin barrier films. Barrier films can be made with thicknesses ranging from 0.5 micron to 20 microns. The films are preferentially less than 30 microns. The film can optionally be pre-treated or post treated with chemical etching, plasma treating, corona treatment, roughening, or other methods known to those of skill in the art to improve bonding to the elastomer body. In some embodiments, the film, e.g., the ePTFE film or densified ePTFE film, surrounds the elastomeric body 125 in its entirety. In other embodiments, the film partially surrounds the elastomeric body 125. For example, the film may only surround a portion of stopper that is not in contact with the fluid or medicament within the syringe.

In other embodiments, the barrier film may comprise a composite fluoropolymer film having a barrier layer and a porous layer. The barrier layer can be formed of densified expanded polytetrafluoroethylene, expanded polytetrafluoroethylene, polytetrafluoroethylene (PTFE), fluorinated ethylene propylene (FEP), polyethylene, polypropylene, polyvinylidene fluoride, polyvinylfluoride, perfluoropropylevinylether, perfluoroalkoxy polymers, and the like. The porous layer can be formed of ePTFE or other porous expanded and advantageously fibrilizing fluoropolymers (for example, ePTFE as taught in U.S. Patent No. 3,953,566 to Gore). The ePTFE layers may advantageously be filled with an organic or inorganic material to provide color, lubricity or other function. In one non-limiting embodiment, the barrier layer is a tri-layer composite including a densified fluoropolymer (e.g., densified ePTFE), thermoplastic adhesive (e.g., FEP, and a porous fluoropolymer (e.g., ePTFE).

The stoppers described herein may be used in syringes for storing and delivering a fluid, typically for medical use. In some embodiments, the syringe is pre-filled with a fluid (e.g., a pre-filled syringe). In one embodiment, the syringes contain a fluid that treats diseases, such as, but not limited to, ocular disease (e.g., macular degeneration or glaucoma) and diabetes. Advantageously, the syringes do not contain a lubricant such as silicone or other liquid lubricant. Thus, the barrels in the syringes described herein are free or substantially free of silicone or other liquid lubricant.

According to the invention, the syringe of the present disclosure includes a barrel which contains a region of reduced diameter located at the proximal end of the barrel to enhance the interference fit between the stopper and the interior surface of the barrel so as to prevent or inhibit oxygen permeation into the barrel. As shown in FIGS. 1a and 1b, the barrel 15 has a region of reduced diameter 16 to enhance the interference fit between the stopper 100 and interior surface of the barrel at the region of reduced diameter 16. As the interference fit increases, so does the resistance to oxygen permeation. To provide an increased interference fit while ensuring adequate minimal depression force for ease of operation, the region of reduced diameter 16 is provided on only a portion of the barrel 15, e.g., adjacent to the proximal end 25 of the barrel 15. This allows one or more ribs 120 on the stopper 100 to reside within the region of reduced diameter 16 for an increased interference fit to inhibit oxygen permeation prior to use. A slight movement of the stopper 100 during depression (*e.g.*, distal advancement of the plunger rod 85) allows one or more ribs 120 to move distally in the barrel 15 beyond the region of reduced diameter 16 and move easily through the barrel 15 to expel the fluid 135.

FIG. 1a also shows a fluid 135 provided in fluid chamber 30 of the barrel 15 of the syringe 10 (e.g., a prefilled syringe). For purposes of illustration only, the fluid 135 is herein identified as a predetermined dose of a fluid, i.e., a therapeutic, a biologic, medicine or drug, however, it should be understood that the fluid 135 could be any type of liquid or material capable of being expelled from a syringe, or the fluid 135 may be absent from the receiving chamber (e.g., an unfilled syringe).

FIG. 1b shows an exploded view of the syringe according to some embodiments. The barrel 15, stopper 100, and plunger rod 85 are three independent and discrete elements that form the syringe 10. One or more of these components work in conjunction to inhibit oxygen permeation into the barrel 15 of the syringe 10 prior to use.

As discussed herein, there are numerous ways to form a region of reduced diameter to reduce the inner diameter of the barrel 15 over a portion of its length. In one embodiment, the barrel 15 of the syringe 10 can be fabricated such that a portion of the barrel has a reduced diameter, as is shown in FIGS. 1a and 1b. For example, a glass syringe may be heated and shaped around a mandrel to set the inner diameter. This heat treatment can be applied to only a portion of the barrel 15 to form the region of reduced diameter 16. In other embodiments, the barrel 15 can have a first region of constant diameter and a second region of constant diameter, where the first region has a smaller diameter than the second region. The two regions may be separated by one or more steps (not shown), or can gradually transition between the two regions. In another embodiment, the region of reduced diameter 16 can have a tapering barrel 15, e.g., having a frustoconical or conical shape, from a region of reduced diameter to a region of larger diameter. In the embodiment shown in FIGS. 1a and 1b, the barrel includes a frustoconical transition section that connects the two regions and provides a gradual transition therebetween. For example, the barrel 15 includes a frustoconical taper located between the region of reduced diameter and a region of greater diameter adjacent to the distal end of the barrel.

In further embodiments, not shown, an insert is in contact with the interior surface of the barrel to form the region of reduced diameter. The insert may be a solid, conformable material that is adhered or bonded to the interior surface of the barrel 15. Alternatively, the insert can be attached to the interior surface of the barrel 15, e.g., via an adhesive, to form the region of reduced diameter 16. Alternatively, the insert may be integrally formed on the interior surface of the barrel 15. As used herein, the term "insert" refers to a member, e.g., a cylindrical member, that is formed separately from the barrel and that is attached or otherwise affixed, e.g., bonded, to the interior surface of the barrel.

In some embodiments, the barrel 15 may include one or more inserts that are adhered to the interior surface of the barrel. The inserts can have varying diameters and lengths, and may be formed of glass, ceramic, metals, or polymers, so long as they are compatible with the material forming the barrel 15 and the contents of the syringe 10. For example, the insert may comprise a polymer tube, e.g., a fluoropolymer, bonded to the interior surface of the barrel 15. The polymer tube can be heated and inflated to bond to the walls of the interior surface of the barrel 15. In some embodiments, the polymer tube that is bonded to the interior surface of the barrel 15 is a solid material and is preferably a gas barrier to inhibit oxygen permeation. In some embodiments, the polymer tube is heat-conformable in order to provide a seal on a portion of the interior surface of the barrel 15. In some embodiments, the polymer tube has a low coefficient of friction to enable the stopper to slide within the barrel 15.

In some embodiments, a laminate that is adhered or bonded to the interior surface of the barrel 15 may be used to form the region of reduced diameter 16. For example, the laminate may be one or more film layer(s) bonded to the interior surface of the barrel 15. The laminate is bonded, or otherwise attached, to the walls of the interior surface of the barrel. In at least one embodiment, the laminate is attached to the interior surface of the barrel 15 using an adhesive. Alternatively, the laminate can be thermoformed on the interior surface of the barrel 15 as described above.

In some embodiments, the laminate may be in the form of a single high strength thin film or multiple thin high strength films formed on the interior surface of the barrel 15. The high strength thin film(s) may comprise any of the materials described herein for the coating material. The thin films may have a thicknesses ranging from about 0.5 µm to about 100 µm, from about 0.5 µm to about 20 µm, or from about 10 µm to about 15 µm. In some embodiments, the films are less than about 30 µm, less than about 20 µm, or less than about 10 µm. The film may be pre-treated or post-treated with chemical etching, plasma treating, corona treatment, roughening, or the like, to improve the interference fit with stopper.

According to the invention, a coating material is provided on a portion of the inner surface of the barrel to form a coated region which, in turn, forms the region of reduced diameter. The coating material 140 may be formed from a liquid coating material 139 that is applied to a portion of the interior surface of the barrel 15. FIGS. 2a-c show a process of coating the barrel 15 according to one aspect of the disclosure. In this process, a portion adjacent to the proximal end 25 of barrel 15 is coated with a liquid coating material 139 to provide a coated region 145. FIG. 2a shows the proximal portion of a barrel 15 of a syringe 10 before the coating process. The barrel 15 has a uniform and constant diameter before coating. Suitable materials for the coating material 140 include, but are not limited to, fluorinated ethylene propylene, expanded fluorinated ethylene propylene, expanded polytetrafluorethylene (ePTFE), epoxy resins, acrylics, and combinations thereof.

As discussed above, FIG. 2a depicts the barrel 15 of the syringe 10 before being submerged into a coating bath 150 containing the liquid coating material 139. In FIG. 2b, the proximal end 25 of the barrel 15 is submerged in the coating bath 150. The barrel 15 is submerged in the coating bath 150 until the liquid coating material 139 adheres to the interior surface of the barrel 15 to form a coated region 145. Any excess liquid coating material 139 may be removed before the liquid coating material 139 solidifies on the interior surface of the barrel 15. Depending on the composition of the coating material 139 used, after being removed from the coating bath 150, the liquid coating substance 139 that has adhered to the interior of the barrel 15 may be dried (such as via air or heat), UV cured, heat cured, or ambient air cured, to form the coating material 140. Alternatively, the liquid coating material 139 can be dried to form the coating material 140. The coating material 140 forms the coated region 145 of the barrel 15 that forms the region of reduced diameter.

It is to be noted that FIG. 2c shows the exterior surface of the barrel 15 without any liquid coating material 139. After the barrel 15 is lifted from the coating bath 150, the liquid coating material 139 is removed from the exterior surface of the barrel 15 before it can solidify or adhere to the exterior surface. For example, the liquid coating material 139 may be blown off the exterior surface of the barrel 15 with deionized air. After the liquid coating material is blown off the exterior surface of the barrel 15, only the interior surface of the barrel 15 includes coating material 140. The coating material 140 on the interior surface of the barrel solidifies to form the coated region 145.

In some embodiments, coating material 140 includes fluorinated ethylene propylene, expanded fluorinated ethylene propylene, epoxy resins, acrylics, and combinations thereof. The coating material 140 may further include an oxygen-absorbing or oxygen-adsorbing material. In some embodiments, the oxygen-absorbing material may include ascorbic acid, sodium ascorbate, activated carbon, and combinations thereof. The oxygen-adsorbing material may include iron, nickel, tin, copper, zinc, tungsten, cobalt, palladium, platinum, zinc oxide, cerium mixed oxides, zirconium mixed oxides, metal oxides (*e.g*., zinc oxide and iron oxide), iron hydroxide, iron carbide, ascorbic acid, sodium ascorbate, catechol, phenol, activated carbon, polymeric materials, metal oxides, e.g., titanium oxide, and combinations thereof The oxygen-absorbing or oxygen-adsorbing material should be compatible with the contents of the syringe. It is to be appreciated that the oxygen-absorbing materials and the oxygen-adsorbing materials can be used alone, or in combination with each other. It is also to be appreciated that the oxygen-adsorbing materials are not limited to the materials listed above, but may include any suitable material that captures or retains oxygen on a surface thereof. Similarly, it is also to be appreciated that the oxygen-absorbing materials are not limited to the materials listed above, but may include any suitable material that adsorbs, adsorbs, or otherwise consumes oxygen. Additional oxygen-absorbing or oxygen-adsorbing materials may include low molecular weight organic compounds such as phenol and polymeric materials incorporating a resin and a catalyst.

In some embodiments, coating material 140 is provided in a region from the flanges 80 to no more than half the length of the barrel 15, e.g., half the length, less than half the length, less than a quarter of the length, or less than an eighth of the length. In one aspect, the coating material 140 is not in contact with the fluid within the fluid chamber 30 when the coating material 140 may contaminate the fluid. In another aspect, the coating material 140 may contact the fluid within the fluid chamber 30 when the coating material 140 would not contaminate the fluid or the coating material 140 is inert.

In another embodiment, during the coating process, the liquid coating material 139 may include a liquid carrier, optionally a solvent, and a coating precursor dissolved, suspended or emulsified therein. The coating precursor optionally is in the form of particles suspended in the liquid carrier. For example, the particle size may be smaller than the thickness of the coating so that the coating is uniform. After application onto the syringe, the liquid carrier is preferably removed, e.g., by heating and/or drying, resulting in the formation of a dry coating material on the syringe. The temperature of the liquid coating material may range from about 25 °C to about 350 °C during the application process. The temperature will of course be dependent on the substrate being coated. Metal, ceramic and glass can withstand elevated temperatures, whereas barrels made of plastic require a lower temperature. In another embodiment, the liquid coating material contains a monomer that can be cured (polymerized), e.g., upon application of UV radiation, to form the solid coating composition on the syringe.

The barrel 15 may be submerged in the coating bath 150 and withdrawn at a rate that facilitates formation of a uniform, defect-free or substantially defect-free coating of a desired thickness on the barrel 15. For example, barrel 15 may be coated with a thin film of a thermoplastic resin of the coating material 140 and then dried or cured to form the coated region 145. A pre-molded shape optionally may be pressed against the coating material 140 on a portion of the interior surface of the barrel 15 to generally conform the interior surface to a desired shape or thickness.

In some embodiments, barrel 15 can be dipped in a liquid coating material 139 followed by heat treatment, curing and/or spin drying. In other embodiments, the coating material may be applied to the syringe by a spray process or by brush application of the liquid coating material 139, followed by drying and/or curing, as described above to form the coated region 145.

FIG. 2c shows a portion of a coated barrel after it has been removed from the coating bath 150. In the embodiment shown in FIG. 2c, a portion of the proximal end 25 of the barrel 15 is coated to provide a coated region 145. As shown, barrel 15 of syringe 10 may have a coated region 145 (formed of the coating material 140) at a proximal end 25 and an uncoated distal end (not depicted) adjacent the coated region 145. The coated region 145 (formed of the coating material 140) has a diameter that is less than the diameter of the uncoated region. For example, the diameter of the coated region 145 may be at least about 25 µm less, at least 50 µm less, or at least 100 µm less, than the diameter of the uncoated region. The smaller diameter caused by the coated region 145 provides for a tighter interference fit with the stopper 100 to inhibit oxygen permeation into the fluid chamber 30 of the syringe. The thickness of the coated region 145 may vary according to many factors, i.e., number of coats of the liquid coating material 139, viscosity of coating, and/or speed of withdrawal during dip process. The coating material 140 may be ground, machined, or melted to a desired thickness. For example, the thickness of the coating material 140 (and thus the coated region 145) may range from about 0.5 µm to about 100 µm, from about 0.5 µm to about 20 µm, or from about 10 µm to about 15 µm. In some embodiments, the coating material 140 (and thus the coated region 145) has a thickness less than about 30 µm, less than about 20 µm, or less than about 10 µm.

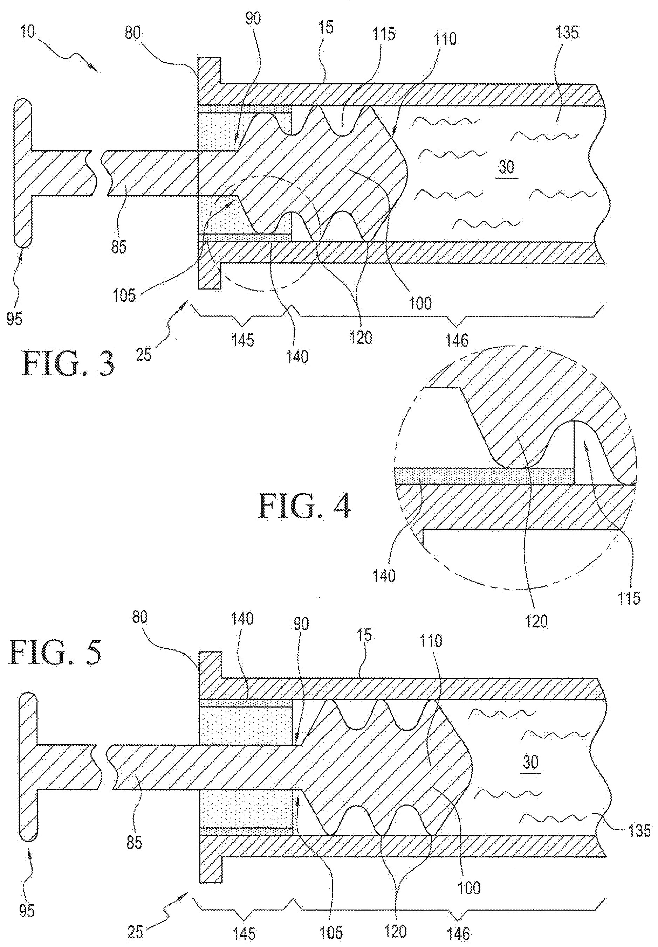

FIG. 3 shows a syringe 10 with a portion of the interior surface of the barrel 15 having thereon a coated region 145 formed of the coating material 140 at a proximal end thereof. In one embodiment, at least one or more ribs 120 of the stopper 100 are located within the coated region 145 of the barrel 150 in an undepressed state. For example, at least one rib 120, at least two ribs, or at least three ribs (or more) of the stopper 100 are disposed within coated region 145 formed of the coating material 140 in an undepressed state. In some embodiments, the entire stopper 100 may be positioned within the coated region 145 while in the undepressed state.

The coating material 140 is located on a portion of the barrel 15 adjacent to the proximal end 25 of barrel 15. The coated region 145 has a diameter that is less than the diameter of the uncoated region of the barrel, as indicated above. As a result, the portion of the stopper 100 within coated region 145 is more compressed than the portion of the stopper 100 within uncoated region 146.

In an undepressed state, one or more of the ribs 120 of the stopper 100 are in abutting contact with the coating material 140 in the coated region 145. The one or more ribs 120 have a first contact area while in abutting contact with the coating material 140 in the coated region 145 and a second contact area, less than the first contact area, while in contact with the uncoated region 146. By "abutting contact" it is meant that the longitudinal edges of the ribs contact an inner wall formed by the region of reduced diameter, e.g., coating region, thereby compressing the ribs.

FIG. 4 shows an exploded view of one of the ribs 120 of stopper 100 shown in FIG. 3. In an undepressed state, the stopper 100 includes at least one rib 120 in abutting contact with the coating material 140 in the coated region 145. The coated region 145 has a smaller diameter than the uncoated region 146 of the barrel 15, which provides an increased interference fit with the ribs 120 of stopper 100 that are in the coating region. The first contact area may be at least 1% greater than the second contact area, e.g., at least 5% greater, or at least 10% greater. Due to the reduced diameter of a portion of the interior surface of the barrel 15, the stopper 100 has an increased interference fit with the barrel 15 in the coated region 145. The increased interference fit in the coated region 145 inhibits or prevents oxygen from permeating into the barrel 15 of syringe 10. In some embodiments, the coated region 145 has an inner diameter that provides a combination of an increased inference fit with minimal slide force.

FIG. 5 shows the syringe in a depressed state, meaning that the plunger 95 has been pushed distally such that the stopper 100 is no longer in abutting contact with coated region 145. That is, in a depressed state, all of the ribs 120 of the stopper 100 are in contact with an uncoated region 146 of the interior surface of barrel 15. In use, the syringe 10 can administer a fluid 135, e.g., a therapeutic, to a patient in need thereof. The method of administering a fluid may begin by loading the fluid chamber 30 with a fluid 135. In some embodiments, the syringe 10 is preloaded with a fluid 135. In the undepressed state, at least one rib 120 of the stopper 100 is in the coated region 145 of the barrel 15. A user can apply a force to depress the plunger rod 85 to move the stopper 100 distally within the syringe barrel 15 and force the fluid 135 through the injection port 35 at distal end of the barrel 15 when the plunger rod 85 is depressed. As shown in FIG. 5, the force moves at least one rib 120 from the coated region 145 into the uncoated region 146 in the depressed state.

Due to the enhanced interference fit of the one or more ribs 120 with the interior surface of the barrel 15 in the coated region 145, an initial force to move the stopper 100 in the coated region 145 may be greater than a force to move the stopper 100 in the uncoated region 146. However, due to the materials used to coat the barrel, the stopper 100 moves smoothly in the coated region 145 without requiring the use of a lubricant such as silicone. For example, the initial force exerted on the plunger rod may be greater to move the stopper within the coated region 145 as compared to moving the stopper while it is entirely within the uncoated region.

It is also contemplated that in some embodiments, the composition of the coating material employed in the coated region 145 may have a lower coefficient of friction than the material forming the barrel. As such, in these embodiments, the initial force may be comparable, e.g., within 5% of, or even less, than the force required to move the stopper over the uncoated region, notwithstanding the reduced diameter of the coated region 145.

In another embodiment, one or more ring members may be provided on the stopper to reduce or inhibit the permeation of oxygen into the barrel of the syringe. As used herein, the term "ring member" refers to any circumferential member configured to extend laterally around the stopper, including but not limited to, any torus-shaped member, *e.g.,* an O-ring, a ring shape, or cylindrical member. It is to be noted that the outer shape, or general volume, of the ring member may be any shape provided that it is a containment for the oxygen absorbing and/or adsorbing materials. In addition, the shape of the ring member is ultimately dependent on the shape of the stopper and the barrel. The shape may also depend on the size/depth of the valley of the stopper. Herein, the ring members are depicted as circular for ease of description.

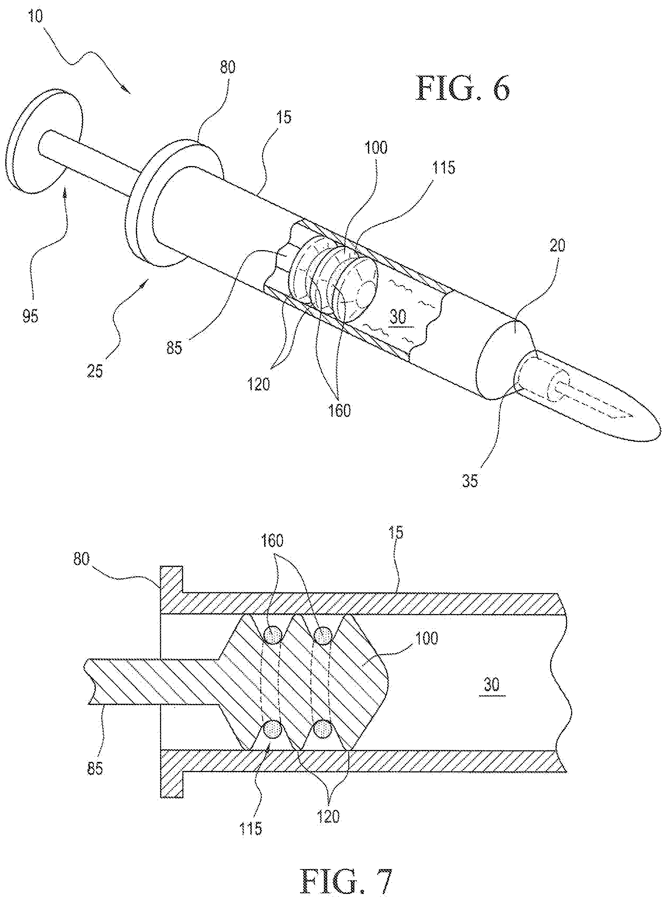

As shown in FIGS. 6 and 7, ring members 160 may be provided in one or more valleys 115 of the stopper 100. In some embodiments, the ring members 160 are in contact with and may tightly wrap around the circumference of the valleys 115 of the stopper 100. The ring members 160 may include an oxygen-absorbing material and/or an oxygen-adsorbing material. The oxygen-absorbing or oxygen-adsorbing material binds and/or otherwise removes oxygen that may enter into the valleys 115 of the stopper 100 before permeating into the fluid chamber 30, thereby inhibiting contamination of any fluids, e.g., oxygen-sensitive drugs or compositions, contained within the barrel. In some embodiments, the ring members 160 may be adhered, affixed, or otherwise attached to one or more valleys 115 of the stopper 100. As used herein, the term "oxygen-sensitive" refers to the ability of a substance to be oxidized or otherwise adversely impacted by coming into contact with oxygen under ambient conditions. In other embodiments, the ring members 160 may include other materials that adsorb or absorb other gaseous substances that may permeate into the syringe.

FIG. 7 shows a cross-sectional view of the syringe of FIG. 6. In this embodiment, ring members 160 are provided in each valley 115 of stopper 100. The ring members 160 may be formed of an elastic or rigid circular body. In the event that oxygen permeates into a valley 115 between a pair of ribs 120, the oxygen-absorbing and/or oxygen-adsorbing material in the ring members 160 inhibit or prevent the oxygen from contaminating the fluid in the fluid chamber 30. Thus, the oxygen-absorbing or oxygen-adsorbing material absorbs, adsorbs, and/or otherwise removes any oxygen from the valleys of the stopper without significantly impacting the slide force. Suitable materials for oxygen-absorbing or oxygen-adsorbing materials may include, but are not limited to, the materials described above.

According to some embodiments, the ring members containing or formed of the oxygen-absorbing and/or oxygen-adsorbing material can be fabricated according to conventional methods, i.e., melt extruded, coated, etc. For example, the ring members may be melt extruded with an evenly dispersed amount of oxygen-absorbing and/or oxygen-adsorbing material. In some embodiments, the ring members may be fabricated into the body of the stopper during manufacture. In some embodiments, the diameter of the ring members is measured and cut to correspond to the circumference of the valleys of the stopper. In other embodiments, a bonding agent may be applied to fix the ring members to each valley.

In some embodiments, to further inhibit oxygen permeation, the stopper 100 including ring members can be provided in a syringe barrel that includes the coated region described above. The coated region is on a portion of the interior surface of the barrel adjacent to the proximal end of the barrel. The coated region forms a region of reduced diameter which has a diameter that is less than the diameter of an uncoated region of the barrel. In this embodiment, the coating region mechanically improves the interference fit between the syringe barrel and the stopper, while the ring members chemically absorb and/or adsorb any oxygen that may permeate into the barrel of the syringe.

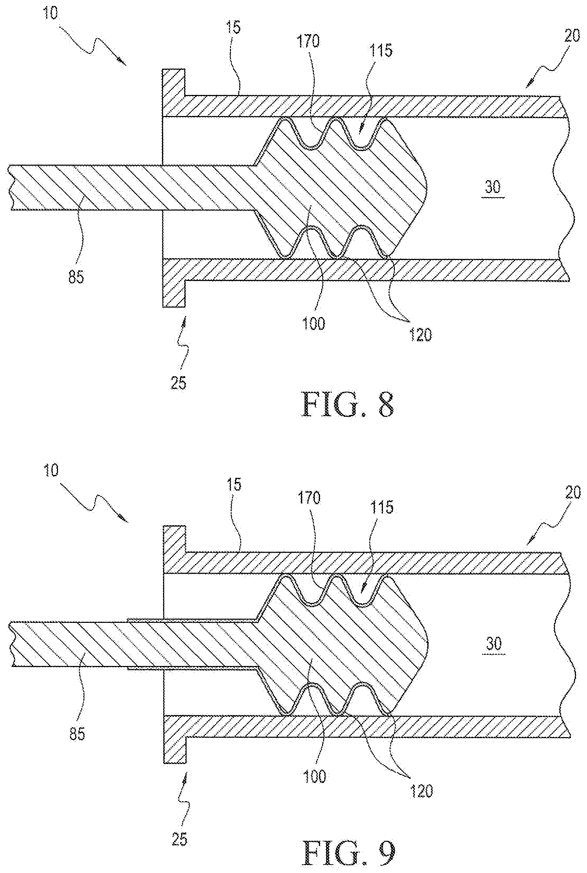

FIG. 8 shows another embodiment where the stopper includes a barrier coating 170 surrounding a portion of the stopper 100. In exemplary embodiments, the barrier coating 170 includes or is formed of an oxygen barrier material that inhibits or prevents oxygen permeation into the barrel without significantly impacting the slide force, i.e., the force to distally move the stopper in the barrel, of the stopper 100 or contaminating fluids within the fluid chamber 30. In some aspects, the oxygen barrier material minimizes friction between the stopper and the interior surface of the barrel as the plunger rod is displaced in the barrel while dispensing a fluid composition. For example, the coefficient of friction of the barrier coating 170 may be less than the coefficient of friction of the stopper material thereby allowing the stopper 100 to move smoothly in the barrel 15. The oxygen barrier material may be selected from the previously described oxygen barrier materials. In addition, the oxygen barrier material may be selected according to its compatibility with the contents of the syringe.

In at least one embodiment, the barrier coating 170 surrounds a portion of the stopper 100 that is not exposed to the fluid chamber 30. As discussed herein, the stopper 100 includes an elastomeric body that has a plurality of ribs 120 and a valley 115 between each pair of adjacent ribs 120. As shown in FIG. 8, the barrier coating 170 is provided on the ribs 120 and the valley 115 between each pair of ribs 120 that are not in contact with the fluid chamber 30.Thus, the barrier coating 170 may not be provided on the distal fluid-contacting surface of stopper 100. It is contemplated, however, that in aspects where the fluid composition in the fluid chamber 30 is not adversely impacted by coming into contact with the barrier coating 170 (e.g., the barrier coating 170 is inert to the fluid or therapeutic in the barrel), that the barrier coating 170 may also cover the distal surface of stopper 100 (e.g, the entirety of the stopper 100).

In some embodiments, the oxygen barrier material is selected from perfluorosulfonated acid, hydrocarbon ionomers, sulfonated polyimides, polyvinyl alcohol, and combinations thereof. Other non-limiting examples of oxygen barrier materials include high density polyethylene (HDPE), low density polyethylene (LDPE), ethylene/vinyl alcohol copolymer (EVOH), polypropylene (PP), polyethylene terephthalate (PET), polyethylene naphthalate (PEN), polyamide (PA), polyvinyl alcohol (PVOH), polyvinyl chloride (PVC), polyvinylidene chloride (PVDC), polychlorotrifluoroethylene (PCTFE), vinylidene, chloride/methyl acrylate copolymer, polyamide, polyester, metalized film, aluminum foil, oxide coated films, and combinations thereof. Metal foil (e.g., aluminum) or SiOx compounds can be used to provide very low permeability to oxygen. Metalized films can include a sputter coating or other application of a metal layer such as aluminum.

Embodiments of the oxygen barrier materials can also or alternatively be present in the form of multilayer films. Multilayer films (e.g., 2, 3, 4, 5 or 6 layer films) may include one or more of the previously described oxygen barrier material(s), and may also include additional layers of non-barrier materials such as PET, polyethylene (PE) and/or coated (e.g., clay, wax, plastic, or the like) or uncoated paper. Suitable multilayer films include, but are not limited to, PVC/EVOH, PET/EVOH, PET/EVOH/PE, PET/EVOH/PET, PE/EVOH/PE, PVC/PCTFE/EVOH, Paper/Aluminum (Alu)/PE, PET/Alu/PE, Paper/PE/foil/PE, Paper/PET/Alu, Clay-coated paper/PE/foil/LDPE, Paper/LDPE/foil/ethylene-ethyl acrylate, and related films thereof. Layers can be bonded together via the use of adhesives (*e.g*., a polyolefin blend (mixture of poly(α-olefins), or polyamide resins). In some embodiments, the barrier coating 170 includes an oxygen barrier material as a multilayer film. For example, the barrier coating 170 may include a laminate that is adhered or bonded to the stopper 100.

In other embodiments, the barrier coating 170 may further include an oxygen-absorbing material or an oxygen-adsorbing material. Suitable materials for oxygen-absorbing or oxygen-adsorbing materials may include, but are not limited to, the materials described above.

FIG. 9 depicts a cross-sectional view of a syringe according to another embodiment. In this embodiment, both the plunger rod 85 and the stopper 100 include the barrier coating 170 that includes an oxygen barrier material that inhibits or prevents the ingress of oxygen into the fluid chamber. For example, a portion of the plunger rod 85 that is attached to the stopper 100 may be located within the barrel 15 of the syringe. In one embodiment, the portion of the plunger rod 85 within the barrel 15 may also include the barrier coating 170 to assist in the prevention of oxygen into the syringe. The barrier coating 170 on the plunger rod 85 may serve as an initial barrier for inhibiting oxygen permeation before reaching the stopper 100.

In some embodiments, the syringe may include any combination of the embodiments described above. For example, it is contemplated that the barrel may include a coating on a portion of the interior surface of the barrel to form a region of reduced diameter; the stopper may include one or more ring members comprising an oxygen-absorbing or oxygen-adsorbing material; and the stopper may include an oxygen barrier material. In this way, the coating mechanically improves the interference fit between the syringe barrel and the stopper, while the ring members chemically absorb or adsorb any oxygen that may permeate into the barrel of the syringe, and the oxygen barrier material on the surface of the stopper prevents the ingress of any oxygen.

The process of coating the stopper with an oxygen barrier material may include dipping a portion of the stopper in a suitable solution containing the above-described oxygen barrier material and/or oxygen-absorbing or oxygen-adsorbing material. In some embodiments, the portion of the stopper that will be in contact with the fluid in a syringe is not dipped in the solution, i.e., it remains inert. The stoppers may be removed from the solution and allowed to fully dry or cure. For example, in one embodiment, clean stoppers may be partially immersed in the solution until the solution adhered to the stopper, then removed from the solution, and excess solution on the stoppers may be blown off with deionized air. The stoppers may then be allowed to dry for about one hour in an atmosphere of approximately 25° C.

In some embodiments, the syringe may include any combination of the embodiments described above. For example, it is contemplated that the barrel may include a coating on a portion of the interior surface of the barrel to form a region of reduced diameter; the stopper may include one or more ring members comprising an oxygen-absorbing or oxygen-adsorbing material; and the stopper may include an oxygen barrier material. In this way, the coating mechanically improves the interference fit between the syringe barrel and the stopper, while the ring members chemically absorb or adsorb any oxygen that may permeate into the barrel of the syringe, and the oxygen barrier material on the surface of the stopper prevents the ingress of any oxygen.

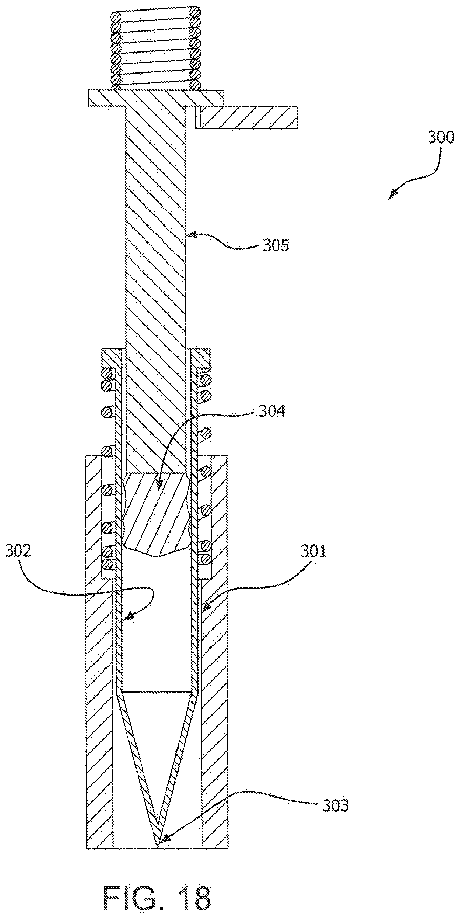

FIG. 18 shows an auto-injector 300 in accordance with some embodiments. The auto-injector 300 includes a barrel 301, an injection member 303 for injecting a drug solution, and a stopper 304. The stopper 304 may be integral with or attached to a plunger rod 305. The inner surface 303 of the barrel 301 may be lubricant-free. It is within the scope of the invention that stopper 304 may include ribs and/or a stopper as depicted and described with respect to FIGS. 10-17, or it may be a stopper without ribs. The auto-injector 300 may incorporate a variable actuation force applied to the stopper 304.

In another aspect, the syringe, described in detail herein, as well as an auto-injector or pen, may be used in combination with different therapeutic compounds such as, for example, drugs and biologics, including but not limited to, antibodies, antisense, RNA interference, gene therapy, primary and embryonic stem cells, vaccines, and combinations thereof. For instance, the embodiments described herein may be utilized in combination with any or all of the following:

Cell therapy using cells that are derived primarily from endoderm such as Exocrine secretory epithelial cells and Hormone-secreting cells; ectoderm such as Keratinizing epithelial cells, Wet stratified barrier epithelial cells, Sensory transducer cells, Autonomic neuron cells, Sense organ and peripheral neuron supporting cells, Central nervous system neurons and glial cells, Lens cells; mesoderm such as Metabolism and storage cells, Barrier function cells (lung, gut, exocrine glands, and urogenital tract), Extracellular matrix cells, Contractile cells, Blood and immune system cells, Germ cells, Nurse cell, Interstitial cells or a combination thereof. Additionally cells that are genetically, chemically or physically altered or modified are considered to be in the scope of the invention.

Examples of Exocrine secretory epithelial cells include, but are not limited to, Salivary gland mucous cell, Salivary gland number 1, Von Ebner's gland cell in tongue, Mammary gland cell, Lacrimal gland cell, Ceruminous gland cell in ear, Eccrine sweat gland dark cell, Eccrine sweat gland clear cell, Apocrine sweat gland cell, Gland of Moll cell in eyelid, Sebaceous gland cell, Bowman's gland cell in nose, Brunner's gland cell in duodenum, Seminal vesicle cell, Prostate gland cell, Bulbourethral gland cell, Bartholin's gland cell, Gland of Littre cell, Uterus endometrium cell, Isolated goblet cell of respiratory and digestive tracts, Stomach lining mucous cell, Gastric gland zymogenic cell, Gastric gland oxyntic cell, Pancreatic acinar cell, Paneth cell of small intestine, Type II pneumocyte of lung, Clara cell of lung; Hormone-secreting cells including but not limited to: Anterior pituitary cells, Intermediate pituitary cell, Magnocellular neurosecretory cells, Gut and respiratory tract cells, Thyroid gland cells, Parathyroid gland cells, Adrenal gland cells, Leydig cell of testes secreting testosterone, Theca interna cell of ovarian follicle secreting estrogen, Corpus luteum cell of ruptured ovarian follicle secreting progesterone, Juxtaglomerular cell, Macula densa cell of kidney, Peripolar cell of kidney, Mesangial cell of kidney, Pancreatic islets; Keratinizing epithelial cells including but not limited to: Epidermal keratinocyte, Epidermal basal cell, Keratinocyte of fingernails and toenails, Nail bed basal cell, Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, Hair matrix cell; Wet stratified barrier epithelial cells including but not limited to: Surface epithelial cell of stratified squamous epithelium and basal cell of epithelia of cornea, tongue, oral cavity, esophagus, anal canal, distal urethra and vagina, Urinary epithelium cell; Sensory transducer cells including but not limited to: Auditory inner hair cell of organ of Corti, Auditory outer hair cell of organ of Corti, Basal cell of olfactory epithelium, Cold-sensitive primary sensory neurons, Heat-sensitive primary sensory neurons, Merkel cell of epidermis, Olfactory receptor neuron, Pain-sensitive primary sensory neurons, Photoreceptor cells of retina in eye: Proprioceptive primary sensory neurons, Touch-sensitive primary sensory neurons, Type I carotid body cell, Type II carotid body cell, Type I hair cell of vestibular system of ear, Type II hair cell of vestibular system of ear, Type I taste bud cell; Autonomic neuron cells including but not limited to: Cholinergic neural cell, Adrenergic neural cell, Peptidergic neural cell; Sense organ and peripheral neuron supporting cells including but not limited to: Inner pillar cell of organ of Corti, Outer pillar cell of organ of Corti, Inner phalangeal cell of organ of Corti, Outer phalangeal cell of organ of Corti, Border cell of organ of Corti, Hensen cell of organ of Corti, Vestibular apparatus supporting cell, Taste bud supporting cell, Olfactory epithelium supporting cell, Schwann cell, Satellite glial cell, Enteric glial cell; Central nervous system neurons and glial cells including but not limited to: Astrocyte, Neuron cells, Oligodendrocyte, Spindle neuron; Lens cells including but not limited to: Anterior lens epithelial cell, Crystallin-containing lens fiber cell; Metabolism and storage cells including but not limited to: Adipocytes: Liver lipocyte; Barrier function cells including but not limited to: Kidney parietal cell, Kidney glomerulus podocyte, Kidney proximal tubule brush border cell, Loop of Henle thin segment cell, Kidney distal tubule cell, Kidney collecting duct cell, Principal cells, Intercalated cells, Type I pneumocyte, Pancreatic duct cell, Nonstriated duct cell, Principal cell, Intercalated cell, Duct cell, Intestinal brush border cell, Exocrine gland striated duct cell, Gall bladder epithelial cell, Ductulus efferens nonciliated cell, Epididymal principal cell, Epididymal basal cell; Extracellular matrix cells including but not limited to: Ameloblast epithelial cell, Planum semilunatum epithelial cell of vestibular system of ear, Organ of Corti interdental epithelial cell, Loose connective tissue fibroblasts, Corneal fibroblasts, Tendon fibroblasts, Bone marrow reticular tissue fibroblasts, Other nonepithelial fibroblasts, Pericyte, Nucleus pulposus cell of intervertebral disc, Cementoblast/cementocyte, Odontoblast/odontocyte, Hyaline cartilage chondrocyte, Fibrocartilage chondrocyte, Elastic cartilage chondrocyte, Osteoblast/osteocyte, Osteoprogenitor cell, Hyalocyte of vitreous body of eye, Stellate cell of perilymphatic space of ear, Hepatic stellate cell, Pancreatic stelle cell; Contractile cells including but not limited to: Skeletal muscle cell, Satellite cell, Heart muscle cells, Smooth muscle cell, Myoepithelial cell of iris, Myoepithelial cell of exocrine glands; Blood and immune system cells including but not limited to: Erythrocyte, Megakaryocyte, Monocyte, Connective tissue macrophage, Epidermal Langerhans cell, Osteoclast, Dendritic cell, Microglial cell, Neutrophil granulocyte, Eosinophil granulocyte, Basophil granulocyte, Hybridoma cell, Mast cell, Helper T cell, Suppressor T cell, Cytotoxic T cell, Natural Killer T cell, B cell, Natural killer cell, Reticulocyte, Stem cells and committed progenitors for the blood and immune system; Germ cells including but not limited to: Oogonium/Oocyte, Spermatid, Spermatocyte, Spermatogonium cell, Spermatozoon; Nurse cell including but not limited to: Ovarian follicle cell, Sertoli cell, Thymus epithelial cell; Interstitial cells including but not limited to: Interstitial kidney cells and a combination thereof.

Examples of antibodies, antisense, RNA interference, or gene therapy made to protein targets or gene(s) of: Ataxia Telangiectasia Mutated, Tumor Protein p53, Checkpoint kinase 2, breast cancer susceptibility protein, Double-strand break repair protein, DNA repair protein RAD50, Nibrin, p53-binding protein, Mediator of DNA damage checkpoint protein, H2A histone family member X, Microcephalin, C-terminal-binding protein 1, Structural maintenance of chromosomes protein 1A; Esterases; Phosphatases; Examples of Ion channels include but are not limited to: ligand-gated ion channels, voltage-gated ion channels; Examples of growth factors include but are not limited to: nerve growth factor (NGF), vascular endothelial growth factor (VEGF), platelet-derived growth factor (PDGF), C-fos-induced growth factor (FIGF), platelet-activating factor (PAF), transforming growth factor beta (TGF-β), b, one morphogenetic proteins (BMPs), Activin, inhibin, fibroblast growth factors (FGFs), granulocyte-colony stimulating factor (G-CSF), granulocyte-macrophage colony stimulating factor (GM-CSF), glial cell line-derived neurotrophic factor (GDNF), growth differentiation factor-9 (GDF9), epidermal growth factor (EGF), transforming growth factor-α (TGF- α), growth factor (KGF), migration-stimulating factor (MSF), hepatocyte growth factor-like protein (HGFLP), hepatocyte growth factor (HGF), hepatoma-derived growth factor (HDGF), Insulin-like growth factors; Examples of G Protein-Coupled Receptors (GPCR) include but are not limited to: Adenosine receptor family, Adrenergic receptor family, Angiotensin II receptor, Apelin receptor, Vasopressin receptor family, Brain-specific angiogenesis inhibitor family, Bradykinin receptor family, Bombesin receptor family, Complement component 3a receptor 1, Complement component 5a receptor 1, Calcitonin receptor family, Calcitonin receptor-like family, Calcium-sensing receptor, Cholecystokinin A receptor (CCK1), Cholecystokinin B receptor (CCK2), Chemokine (C-C motif) receptor family, Sphingosine 1-phosphate receptor family, Succinic receptor, Cholinergic receptor family. Chemokine-like receptor family, Cannabinoid receptor family, Corticotropin releasing hormone receptor family, prostaglandin D2 receptor, Chemokine C-X3-C receptor family, Chemokine (C-X-C motif) receptor family, Burkitt lymphoma receptor, Chemokine (C-X-C motif) receptor family, Cysteinyl leukotriene receptor 2 (CYSLT2), chemokine receptor (FY), Dopamine receptor family, G protein-coupled receptor 183 (GPR183), Lysophosphatidic acid receptor family, Endothelin receptor family, Coagulation factor II (thrombin) receptor family, Free fatty acid receptor family, Formylpeptide receptor family, Follicle stimulating hormone receptor (FSHR), gamma-aminobutyric acid (GABA) B receptor, Galanin receptor family, Glucagon receptor, Growth hormone releasing hormone receptor (GHRH), Ghrelin receptor (ghrelin), Growth hormone secretagogue receptor 1b (GHSR1b), Gastric inhibitory polypeptide receptor (GIP), Glucagon-like peptide receptor family, Gonadotropin-releasing hormone receptor (GnRH), pyroglutamylated RFamide peptide receptor (QRFPR), G protein-coupled bile acid receptor 1 (GPBA), Hydroxycarboxylic acid receptor family, Lysophosphatidic acid receptor 4 (LPA4) Lysophosphatidic acid receptor 5 (GPR92), G protein-coupled receptor 79 pseudogene (GPR79), Hydroxycarboxylic acid receptor 1 (HCA1), G-protein coupled receptor (C5L2, FFA4, FFA4, FFA4, GPER, GPR1, GPR101, GPR107, GPR119, GPR12, GPR123, GPR132, GPR135, GPR139, GPR141, GPR142, GPR143, GPR146, GPR148, GPR149, GPR15, GPR150, GPR151, GPR152, GPR157, GPR161, GPR162, GPR17, GPR171, GPR173, GPR176, GPR18, GPR182, GPR20, GPR22, GPR25, GPR26, GPR27, GPR3, GPR31, GPR32, GPR35, GPR37L1, GPR39, GPR4, GPR45, GPR50, GPR52, GPR55, GPR6, GPR61, GPR65, GPR75, GPR78, GPR83, GPR84, GPR85, GPR88, GPR97, TM7SF1), Metabotropic glutamate receptor family, Gastrin releasing peptide receptor (BB2), Orexin receptor family, Histamine receptor family, 5-hydroxytryptamine receptor family, KISS1-derived peptide receptor (kisspeptin), Leucine-rich repeat-containing G protein-coupled receptor family, horiogonadotropin receptor (LH), Leukotriene B4 receptor (BLT1), Adenylate Cyclase Activating Polypeptide 1 Receptor 1 (mPAC1), Motilin receptor, Melanocortin receptor family, Melanin concentrating hormone receptor 1 (MCH1), Neuropeptide Y1 receptor (Y1), Neuropeptide Y2 receptor (NPY2R), Opioid receptor family, Oxytocin recepter (OT), P2Y Purinoceptor 12 (mP2Y12), P2Y Purinoceptor 6 (P2Y6), Pancreatic polypeptide receptor family, Platelet-activating factor receptor family, Prostaglandin E receptor family, Prostanoid IP1 receptor (IP1), MAS-related GPR, member family, Rhodopsin (Rhodopsin), Relaxin family peptide receptor family, Somatostatin receptor family, Tachykinin receptor family, Melatonin receptor family, Urotensin receptor family, Vasoactive intestinal peptide receptor 1 (mVPAC1), Neuromedin B Receptor (BB1), Neuromedin U receptor 1 (NMU1), Neuropeptides B/W receptor family, Neuropeptide FF receptor 1 (NPFF1), neuropeptide S receptor 1 (NPS receptor), Neuropeptide Y receptor family, Neurotensin receptor 1 (NTS1), Opsin 5 (OPN5), Opioid receptor-like receptor (NOP), Oxoeicosanoid (OXE) receptor 1 (OXE), Oxoglutarate (alpha-ketoglutarate) receptor 1 (OXGR1), Purinergic receptor family, Pyrimidinergic receptor family, Prolactin releasing hormone receptor (PRRP), Prokineticin receptor family, Platelet activating receptor (PAF), Prostaglandin F receptor family, Prostaglandin I2 (prostacyclin) receptor family, Parathyroid hormone receptor family, muscarinic 4 (rM4), Prostanoid DP2 receptor (rGPR44), Prokineticin receptor family, Relaxin family peptide receptor family, Secretin receptor (secretin), Smoothened, Frizzled class receptor (Smoothened), trace amine associated receptor family, Tachykinin family, Thromboxane A2 receptor (TP), Thyrotropin-releasing hormone receptor (TRH1), Thyroid Stimulating Hormone Receptor (TSH); Examples of Protein kinases include but are not limited to: AP2 associated kinase, Homo sapiens ABL proto-oncogene 1 - non-receptor tyrosine-protein kinase family, c-abl oncogene 1 receptor tyrosine kinase family, v-abl Abelson murine leukemia viral oncogene homolog 2, activin A receptor family, chaperone - ABC1 activity of bc1 complex homolog (S. pombe) (ADCK3), aarF domain containing kinase 4 (ADCK4), v-akt murine thymoma viral oncogene homolog family, anaplastic lymphoma receptor tyrosine kinase family, protein kinase A family, protein kinase B family, ankyrin repeat and kinase domain containing 1 (ANKK1), NUAK family - SNF1-like kinase, mitogen-activated protein kinase family aurora kinase A (AURKA), aurora kinase B (AURKB), aurora kinase C (AURKC), AXL receptor tyrosine kinase (AXL), BMP2 inducible kinase (BIKE), B lymphoid tyrosine kinase (BLK), bone morphogenetic protein receptor family, BMX non-receptor tyrosine kinase (BMX), v-raf murine sarcoma viral oncogene homolog B1 (BRAF), protein tyrosine kinase 6 (BRK), BR serine/threonine kinase family, Bruton agammaglobulinemia tyrosine kinase (BTK), calcium/calmodulin-dependent protein kinase family, cyclin-dependent kinase family, cyclin-dependent kinase-like family, CHK1 checkpoint homolog (S. pombe) (CHEK1), CHK2 checkpoint homolog (S. pombe) (CHEK2), Insulin receptor, isoform A (INSR), Insulin receptor, isoform B (INSR), rho-interacting serine/threonine kinase (CIT), v-kit Hardy-Zuckerman 4 feline sarcoma viral oncogene homolog (KIT), CDC-Like Kinase family - Hepatocyte growth factor receptor (MET), Proto-oncogene tyrosine-protein kinase receptor, colony-stimulating factor family receptor, c-src tyrosine kinase (CSK), casein kinase family, megakaryocyte-associated tyrosine kinase (CTK), death-associated protein kinase family, doublecortin-like kinase family, discoidin domain receptor tyrosine kinase, dystrophia myotonica-protein kinase (DMPK), dual-specificity tyrosine-(Y)-phosphorylation regulated kinase family, epidermal growth factor receptor family, eukaryotic translation initiation factor 2-alpha kinase 1 (EIF2AK1), EPH receptor family, Ephrin type-A receptor family, Ephrin type-B receptor family, v-erb-b2 erythroblastic leukemia viral oncogene homolog family, mitogen-activated protein kinase family, endoplasmic reticulum to nucleus signaling 1 (ERN1), PTK2 protein tyrosine kinase 2 (FAK), fer (fps/fes related) tyrosine kinase (FER). feline sarcoma oncogene (FES), Fibroblast growth factor receptor family, Gardner-Rasheed feline sarcoma viral (v-fgr) oncogene homolog (FGR), fms-related tyrosine kinase family, Fms-related tyrosine kinase family, fyn-related kinase (FRK), FYN oncogene related to SRC, cyclin G associated kinase (GAK), eukaryotic translation initiation factor 2 alpha kinase, Growth hormone receptor. G protein-coupled receptor kinase 1 (GRK1), G protein-coupled receptor kinase family, glycogen synthase kinase family, germ cell associated 2 (haspin) (HASPIN), Hemopoietic cell kinase (HCK), homeodomain interacting protein kinase family, mitogen-activated protein kinase kinase kinase kinase family, hormonally up-regulated Neu-associated kinase (HUNK), intestinal cell (MAK-like) kinase (ICK), Insulin-like growth factor 1 receptor (IGF1R), conserved helix-loop-helix ubiquitous kinase (IKK-alpha), inhibitor of kappa light polypeptide gene enhancer in B-cells - kinase beta family, insulin receptor (INSR), insulin receptor-related receptor (INSRR), interleukin-1 receptor-associated kinase family, IL2-inducible T-cell kinase (ITK), Janus kinase family, Kinase Insert Domain Receptor, v-kit Hardy-Zuckerman 4 feline sarcoma viral oncogene homolog, lymphocyte-specific protein tyrosine kinase (LCK), LIM domain kinase family, serine/threonine kinase family leucine-rich repeat kinase family, v-yes-1 Yamaguchi sarcoma viral related oncogene homolog (LYN), male germ cell-associated kinase (MAK),, MAP/microtubule affinity-regulating kinase family, microtubule associated serine/threonine kinase family, maternal embryonic leucine zipper kinase, c-mer proto-oncogene tyrosine kinase (MERTK), met proto-oncogene (hepatocyte growth factor receptor), MAP kinase interacting serine/threonine kinase family, myosin light chain kinase family, mixed lineage kinase domain-like protein isoform, CDC42 binding protein kinase family, serine/threonine kinase family, macrophage stimulating 1 receptor (c-met-related tyrosine kinase) (MST1R), mechanistic target of rapamycin (serine/threonine kinase) (MTOR), muscle- skeletal- receptor tyrosine kinase (MUSK), myosin light chain kinase family, NIMA (never in mitosis gene a)-related kinase family, serine/threonine-protein kinase NIM1 (NIM1), nemo-like kinase (NLK), oxidative-stress responsive 1 (OSR1), p21 protein (Cdc42/Rac)-activated kinase family, PAS domain containing serine/threonine kinase, Platelet-derived growth factor receptor family, 3-phosphoinositide dependent protein kinase-1 (PDPK1), Calcium-dependent protein kinase 1, phosphorylase kinase gamma family, Phosphatidylinositol 4,5-bisphosphate 3-kinase, phosphoinositide-3-kinase family, phosphatidylinositol 4-kinase family. phosphoinositide kinase, FYVE finger containing, Pim-1 oncogene (PIM1), pim-2 oncogene (PIM2), pim-3 oncogene (PIM3), phosphatidylinositol-4-phosphate 5-kinase family, phosphatidylinositol-5-phosphate 4-kinase family protein kinase, membrane associated tyrosine/threonine 1 (PKMYT1), protein kinase N family, polo-like kinase family, protein kinase C family, protein kinase D family, cGMP-dependent protein kinase family, eukaryotic translation initiation factor 2-alpha kinase 2 (PRKR), X-linked protein kinase (PRKX), Prolactin receptor (PRLR), PRP4 pre-mRNA processing factor 4 homolog B (yeast) (PRP4), PTK2B protein tyrosine kinase 2 beta (PTK2B), SIK family kinase 3 (QSK), v-raf-1 murine leukemia viral oncogene homolog 1 (RAF1), Neurotrophic tyrosine kinase receptor type family, receptor (TNFRSF)-interacting serine-threonine kinase family, dual serine/threonine and tyrosine protein kinase (RIPK5), Rho-associated, coiled-coil containing protein kinase family, c-ros oncogene 1 , receptor tyrosine kinase (ROS1), ribosomal protein S6 kinase family, SH3-binding domain kinase 1 (SBK1), serum/glucocorticoid regulated kinase family, Putative uncharacterized serine/threonine-protein kinase (Sugen kinase 110) (SgK110), salt-inducible kinase family, SNF related kinase (SNRK), src-related kinase , SFRS protein kinase family, , Spleen tyrosine kinase (SYK), TAO kinase family,, TANK-binding kinase 1 (TBK1), tec protein tyrosine kinase (TEC), testis-specific kinase 1 (TESK1), transforming growth factor, beta receptor family, tyrosine kinase with immunoglobulin-like and EGF-like domains 1 (TIE1), TEK tyrosine kinase, endothelial (TIE2), Angiopoietin-1 receptor (Tie2), tousled-like kinase family, TRAF2 and NCK interacting kinase (TNIK), non-receptor tyrosine kinase family, TNNI3 interacting kinase (TNNI3K), transient receptor potential cation channel, testis-specific serine kinase family, TTK protein kinase (TTK), TXK tyrosine kinase (TXK), Tyrosine kinase 2 (TYK2), TYROS protein tyrosine kinase (TYRO3), unc-51-like kinase family, phosphatidylinositol 3-kinase, vaccinia related kinase 2 (VRK2), WEE1 homolog family, WNK lysine deficient protein kinase family, v-yes-1 Yamaguchi sarcoma viral oncogene homolog 1 (YES), sterile alpha motif and leucine zipper containing kinase AZK (ZAK), zeta-chain (TCR) associated protein kinase 70kDa (ZAP70); Examples of nuclear hormone receptors include but are not limited to: Androgen receptor (AR), Estrogen related receptor alpha (ESRRA), Estrogen receptor 1 (ESR1), Nuclear receptor subfamily 1 - group H - member 4 (NR1H4), Nuclear receptor subfamily 3 - group C - member 1 (glucocorticoid receptor) (NR3C1), Nuclear receptor subfamily 1 - group H - member 3 (Liver X receptor α) (NR1H3), Nuclear receptor subfamily 1 - group H - member 2 (Liver X receptor β) (NR1H2), Nuclear receptor subfamily 1 - group H - member 2 (Liver X receptor β) (NR1H2), Nuclear receptor subfamily 3 - group C - member 2 (Mineralcorticoid receptor) (NR3C2), Peroxisome Proliferator Activated Receptor alpha (PPARA), Peroxisome Proliferator Activated Receptor gamma (PPARG), Peroxisome Proliferator Activated Receptor delta (PPARD), Progesterone receptor α (PGR), Progesterone receptor β (PGR), Retinoic acid receptor - alpha (RARA), Retinoic acid receptor - beta (RARB), Retinoid X receptor - alpha (RXRA), Retinoid X receptor - gamma (RXRG), Thyroid hormone receptor - alpha (THRA), Thyroid hormone receptor - beta (THRB), Retinoic acid-related orphan receptor, Liver X receptor, Farnesoid X receptor, Vitamin D receptor, Pregnane X receptor, Constitutive androstane receptor, Hepatocyte nuclear factor 4, Oestrogen receptor, Oestrogen-related receptor, Glucocortioic receptor, Nerve growth factor-induced-B, Germ cell nuclear factor, Examples of Epigenetic targets include but are not limited to: ATPase family AAA domain-containing protein 2 (ATAD2A), ATPase family - AAA domain containing 2B (ATAD2B), ATPase family AAA domain containing - 2B (ATAD2B), bromodomain adjacent to zinc finger domain - 1A (BAZ1A), bromodomain adjacent to zinc finger domain - 1B (BAZ1B), bromodomain adjacent to zinc finger domain - 2A (BAZ2A), bromodomain adjacent to zinc finger domain - 2A (BAZ2A), bromodomain adjacent to zinc finger domain - 2B (BAZ2B), bromodomain-containing protein 1 (BRD1), Bromodomain containing protein 2 - 1st bromodomain (BRD2), Bromodomain containing protein 2 - 1st & 2nd bromodomains (BRD2), bromodomain-containing protein 2 isoform 1 - bromodomain 2 (BRD2(2)), bromodomain-containing protein 3 - bromodomain 1 (BRD3(1)), Bromodomain-containing protein 3 - 1st bromodomain (BRD3), Bromodomain-containing protein 3-1st & 2nd bromodomains (BRD3), bromodomain-containing protein 3 - bromodomain 2 (BRD3(2)), Bromodomain containing protein 4 - 1st bromodomain (BRD4), bromodomain-containing protein 4 isoform long - bromodomains 1 and 2 (BRD4(1-2)), bromodomain-containing protein 4 isoform long - bromodomain 2 (BRD4(2)), bromodomain-containing protein 4 isoform short (BRD4(full-length -short-iso.)), Bromodomain containing protein 7 (BRD7), bromodomain containing 8-bromodomain 1 (BRD8(1)), bromodomain containing 8 - bromodomain 2 (BRD8(2)), bromodomain-containing protein 9 isoform 1 (BRD9), Bromodomain containing testis-specific - 1st bromodomain (BRDT), Bromodomain containing testis-specific - 1st & 2nd bromodomains (BRDT), bromodomain testis-specific protein isoform b - bromodomain 2 (BRDT(2)), bromodomain and PHD finger containing - 1 (BRPF1), bromodomain and PHD finger containing - 3 (BRPF3), bromodomain and PHD finger containing - 3 (BRPF3), Bromodomain and WD repeat-containing 3 - 2nd bromodomain (BRWD3(2)), Cat eye syndrome critical region protein 2 (CECR2), CREB binding protein (CREBBP), E1A binding protein p300 (EP300), EP300 (EP300), nucleosome-remodeling factor subunit BPTF isoform 1 (FALZ), Nucleosome-remodeling factor subunit BPT (FALZ), Euchromatic histone-lysine N-methyltransferase 2 (EHMT2), Histone Acetyltransferase - KAT2A (GCN5L2), Euchromatic histone-lysine N-methyltransferase 1 (EHMT1), Histone-lysine N-methyltransferase MLL (MLL), Polybromo 1 - 1st bromodomain (PB1(1)), Polybromo 1 - 2nd bromodomain (PB1(2)), polybromo 1 - bromodomain 2 (PBRM1(2)), polybromo 1 - bromodomain 5 (PBRM1(5)), Histone acetyltransferase KAT2B (PCAF), PH-interacting protein - 1st bromodomain (PHIP(1)), PH-interacting protein-2nd bromodomain (PHIP(2)), Protein kinase C-binding protein 1 (PRKCBP1), Protein arginine N-methyltransferase 3 (PRMT3), SWI/SNF related - matrix associated - actin dependent regulator of chromatin - subfamily a - member 2 (SMARCA2), SWI/SNF related - matrix associated - actin dependent regulator of chromatin - subfamily a-member 4 (SMARCA4), Nuclear body protein - SP110 (SP110), Nuclear body protein - SP140 (SP140), Transcription initiation factor TFIID subunit 1 (TAF1(1 -2)), TAF1 RNA polymerase II - TATA box binding protein (TBP)-associated factor - 250kDa-bromodomain 2 (TAF1(2)), Transcription initiation factor TFIID subunit 1-like - 1st bromodomain (TAF1L(1)), Transcription initiation factor TFIID subunit 1-like - 2nd bromodomain (TAF1L(2)), tripartite motif containing 24 (TRIM24(Bromo.)), tripartite motif containing 24 (TRIM24(PHD -Bromo.)), E3 ubiquitin-protein ligase TRIM33 (TRIM33), tripartite motif containing 33 (TRIM33(PHD -Bromo.)), WD repeat 9 - 1st bromodomain (WDR9(1)), WD repeat 9 - 2nd bromodomain (WDR9(2)); membrane transport proteins including but not limited to ATP-binding cassette (ABC) superfamily, solute carrier (SLC) superfamily, multidrug resistance protein 1 (P-glycoprotein), organic anion transporter 1,and protein such as EAAT3, EAAC1, EAAT1, GLUT1, GLUT2, GLUT9, GLUT10, rBAT, AE1, NBC1, KNBC, CHED2, BTR1, NABC1, CDPD, SGLT1, SGLT2, NIS, CHT1, NET, DAT, GLYT2, CRTR, B0AT1, SIT1, XT3, y+LAT1, BAT1, NHERF1, NHE6, ASBT, DMT1, DCT1, NRAMP2, NKCC2, NCC, KCC3, NACT, MCT1, MCT8, MCT12, SLD, VGLUT3, THTR1, THTR2, PIT2, GLVR2, OCTN2, URAT1, NCKX1, NCKX5, CIC, PiC, ANT1, ORNT1, AGC1, ARALAR, Citrin, STLN2, aralar2, TPC, MUP1, MCPHA, CACT, GC1, PHC, DTD, CLD, DRA, PDS, Prestin, TAT1, FATP4, ENT3, ZnT2, ZnT10, AT1, NPT2A, NPT2B, HHRH, CST, CDG2F, UGAT, UGTL, UGALT, UGT1, UGT2, FUCT1, CDG2C, NST, PAT2, G6PT1, SPX4, ZIP4, LIV4, ZIP13, LZT-Hs9, FPN1, MTP1, IREG1, RHAG, AIM1, PCFT, FLVCR1, FLVCR2, RFT1, RFT2, RFT3, OATP1B1, OATP1B3, OATP2A1; structural proteins including but not limited to tubulin, heat shock protein, Microtubule-stabilizing proteins, Oncoprotein 18, stathmin, kinesin-8 and kinesin-14 family, Kip3, Kif18A; proteases including but not limited ADAM (a disintegrin and metalloprotease) family; Other molecule targets in signal transductions include but are not limited to: Cell division cycle 25 homolog A (CDC25A), forkhead box O3 (forkhead box O3), nuclear factor of kappa light polypeptide gene enhancer in B-cells inhibitor, alpha (NFKBIA), nuclear factor (erythroid-derived 2)-like 2 (NFE2L2), Natriuretic peptide receptor A (NPR1), Tumor necrosis factor receptor superfamily, member 11a (TNFRSF11A), v-rel reticuloendotheliosis viral oncogene homolog A (avian) (RELA), Sterol regulatory element binding transcription factor 2 (SREBF2), CREB regulated transcription coactivator 1 (CRTC1), CREB regulated transcription coactivator 2 (CRTC2), X-box binding protein 1 (XBP1), Catenin (cadherin-associated protein), beta 1 (CTNNB1), and combinations thereof.

Examples of known biologics include, but are not limited to: Abbosynagis, Abegrin, Actemra, AFP-Cide, Antova, Arzerra, Aurexis, Avastin, Benlysta, Bexxar, Blontress, Bosatria, Campath, CEA-Cide, CEA-Scan, Cimzia, Cyramza, Ektomab, Erbitux, FibriScint, Gazyva, Herceptin, hPAM4-Cide, HumaSPECT, HuMax-CD4, HuMax-EGFr, Humira, HuZAF, Hybri-ceaker, Ilaris, Indimacis-125, Kadcyla, Lemtrada, LeukArrest, LeukoScan, Lucentis, Lymphomun, LymphoScan, LymphoStat-B, MabThera, Mycograb, Mylotarg, Myoscint, NeutroSpec, Numax, Nuvion, Omnitarg, Opdivo, Orthoclone OKT3, OvaRex, Panorex, Prolia, Prostascint, Raptiva, Remicade, Removab, Rencarex, ReoPro, Rexomun, Rituxan, RoActemra, Scintimun, Simponi, Simulect, Soliris, Stelara, Synagis, Tactress, Theracim, Theragyn, Theraloc, Tysabri, Vectibix, Verluma, Xolair, Yervoy, Zenapax, and Zevalin or combinations thereof.

Examples of known monoclonal antibodies include but are not limited to: 3F8, 8H9, Abagovomab, Abciximab, Abituzumab, Abrilumab, Actoxumab, Adalimumab, Adecatumumab, Aducanumab, Afasevikumab, Afelimomab, Afutuzumab, Alacizumab pegol, ALD518, ALD403, Alemtuzumab, Alirocumab, Altumomab pentetate, Amatuximab, AMG 334, Anatumomab mafenatox, Anetumab ravtansine, Anifrolumab, Anrukinzumab, Apolizumab, Arcitumomab, Ascrinvacumab, Aselizumab, Atezolizumab, Atinumab, Atlizumab, Atorolimumab, Avelumab, Bapineuzumab, Basiliximab, Bavituximab, Bectumomab, Begelomab, Belimumab, Benralizumab, Bertilimumab, Besilesomab, Bevacizumab, Bezlotoxumab, Biciromab, Bimagrumab, Bimekizumab, Bivatuzumab mertansine, Bleselumab, Blinatumomab, Blontuvetmab, Blosozumab, Bococizumab, Brazikumab, Brentuximab vedotin, Briakinumab, Brodalumab, Brolucizumab, Brontictuzumab, Burosumab, Cabiralizumab, Canakinumab, Cantuzumab mertansine, Cantuzumab ravtansine, Caplacizumab, Capromab pendetide, Carlumab, Carotuximab, Catumaxomab, cBR96-doxorubicin immunoconjugate, Cedelizumab, Cergutuzumab amunaleukin, Certolizumab pegol, Cetuximab, Citatuzumab bogatox, Cixutumumab, Clazakizumab, Clenoliximab, Clivatuzumab tetraxetan, Codrituzumab, Coltuximab ravtansine, Conatumumab, Concizumab, CR6261, Crenezumab, Crotedumab, Dacetuzumab, Daclizumab, Dalotuzumab, Dapirolizumab pegol, Daratumumab, Dectrekumab, Demcizumab, Denintuzumab mafodotin, Denosumab, Depatuxizumab mafodotin, Derlotuximab biotin, Detumomab, Dinutuximab, Diridavumab, Domagrozumab, Dorlimomab aritox, Drozitumab, Duligotumab, Dupilumab, Durvalumab, Dusigitumab, Ecromeximab, Eculizumab, Edobacomab, Edrecolomab, Efalizumab, Efungumab, Eldelumab, Elgemtumab, Elotuzumab, Elsilimomab, Emactuzumab, Emibetuzumab, Emicizumab, Enavatuzumab, Enfortumab vedotin, Enlimomab pegol, Enoblituzumab, Enokizumab, Enoticumab, Ensituximab, Epitumomab cituxetan, Epratuzumab, Erenumab, Erlizumab, Ertumaxomab, Etaracizumab, Etrolizumab, Evinacumab, Evolocumab, Exbivirumab, Fanolesomab, Faralimomab, Farletuzumab, Fasinumab, FBTA05, Felvizumab, Fezakinumab, Fibatuzumab, Ficlatuzumab, Figitumumab, Firivumab, Flanvotumab, Fletikumab, Fontolizumab, Foralumab, Foravirumab, Fresolimumab, Fulranumab, Futuximab, Galcanezumab, Galiximab, Ganitumab, Gantenerumab, Gavilimomab, Gemtuzumab ozogamicin, Gevokizumab, Girentuximab, Glembatumumab vedotin, Golimumab, Gomiliximab, Guselkumab, Ibalizumab, Ibritumomab tiuxetan, Icrucumab, Idarucizumab, Igovomab, IMA-638, IMAB362, Imalumab, Imciromab, Imgatuzumab, Inclacumab, Indatuximab ravtansine, Indusatumab vedotin, Inebilizumab, Infliximab, Inolimomab, Inotuzumab ozogamicin, Intetumumab, Ipilimumab, Iratumumab, Isatuximab, Itolizumab, Ixekizumab, Keliximab, Labetuzumab, Lambrolizumab, Lampalizumab, Lanadelumab, Landogrozumab, Laprituximab emtansine, LBR-101/PF0442g7429, Lebrikizumab, Lemalesomab, Lendalizumab, Lenzilumab, Lerdelimumab, Lexatumumab, Libivirumab, Lifastuzumab vedotin, Ligelizumab, Lilotomab satetraxetan, Lintuzumab, Lirilumab, Lodelcizumab, Lokivetmab, Lorvotuzumab mertansine, Lucatumumab, Lulizumab pegol, Lumiliximab, Lumretuzumab, LY2951742, Mapatumumab, Margetuximab, Maslimomab, Matuzumab, Mavrilimumab, Mepolizumab, Metelimumab, Milatuzumab, Minretumomab, Mirvetuximab soravtansine, Mitumomab, Mogamulizumab, Monalizumab, Morolimumab, Motavizumab, Moxetumomab pasudotox, Muromonab-CD3, Nacolomab tafenatox, Namilumab, Naptumomab estafenatox, Naratuximab emtansine, Narnatumab, Natalizumab, Navicixizumab, Navivumab, Nebacumab, Necitumumab, Nemolizumab, Nerelimomab, Nesvacumab, Nimotuzumab, Nivolumab, Nofetumomab merpentan, Obiltoxaximab, Obinutuzumab, Ocaratuzumab, Ocrelizumab, Odulimomab, Ofatumumab, Olaratumab, Olokizumab, Omalizumab, Onartuzumab, Ontuxizumab, Opicinumab, Oportuzumab monatox, Oregovomab, Orticumab, Otelixizumab, Otlertuzumab, Oxelumab, Ozanezumab, Ozoralizumab, Pagibaximab, Palivizumab, Pamrevlumab, Panitumumab, Pankomab, Panobacumab, Parsatuzumab, Pascolizumab, Pasotuxizumab, Pateclizumab, Patritumab, Pembrolizumab, Pemtumomab, Perakizumab, Pertuzumab, Pexelizumab, Pidilizumab, Pinatuzumab vedotin, Pintumomab, Placulumab, Plozalizumab, Pogalizumab, Polatuzumab vedotin, Ponezumab, Prezalizumab, Priliximab, Pritoxaximab, Pritumumab, PRO 140, Quilizumab, Racotumomab, Radretumab, Rafivirumab, Ralpancizumab, Ramucirumab, Ranibizumab, Raxibacumab, Refanezumab, Regavirumab, Reslizumab, Rilotumumab, Rinucumab, Risankizumab, Rituximab, Rivabazumab pegol, Robatumumab, Roledumab, Romosozumab, Rontalizumab, Rovalpituzumab tesirine, Rovelizumab, Ruplizumab, Sacituzumab govitecan, Samalizumab, Sapelizumab, Sarilumab, Satumomab pendetide, Secukinumab, Seribantumab, Setoxaximab, Sevirumab, SGN-CD19A, SGN-CD33A, Sibrotuzumab, Sifalimumab, Siltuximab, Simtuzumab, Siplizumab, Sirukumab, Sofituzumab vedotin, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tamtuvetmab, Tanezumab, Taplitumomab paptox, Tarextumab, Tefibazumab, Telimomab aritox, Tenatumomab, Teneliximab, Teplizumab, Teprotumumab, Tesidolumab, Tetulomab, Tezepelumab, TGN1412, Ticilimumab, Tigatuzumab, Tildrakizumab, Timolumab, Tisotumab vedotin, TNX-650, Tocilizumab, Toralizumab, Tosatoxumab, Tositumomab, Tovetumab, Tralokinumab, Trastuzumab, Trastuzumab emtansine, TRBS07, Tregalizumab, Tremelimumab, Trevogrumab, Tucotuzumab celmoleukin, Tuvirumab, Ublituximab, Ulocuplumab, Urelumab, Urtoxazumab, Ustekinumab, Utomilumab, Vadastuximab talirine, Vandortuzumab vedotin, Vantictumab, Vanucizumab, Vapaliximab, Varlilumab, Vatelizumab, Vedolizumab, Veltuzumab, Vepalimomab, Vesencumab, Visilizumab, Vobarilizumab, Volociximab, Vorsetuzumab mafodotin, Votumumab, Xentuzumab, Zalutumumab, Zanolimumab, Zatuximab, Ziralimumab, and Zolimomab aritox or combinations thereof.

Examples of vaccines developed for viral diseases include but are not limited to: Hepatitis A vaccine, Hepatitis B vaccine, Hepatitis E vaccine, HPV vaccine, Influenza vaccine, Japanese encephalitis vaccine, MMR vaccine, MMRV vaccine, Polio vaccine, Rabies vaccine, Rotavirus vaccine, Varicella vaccine, Shingles vaccine, Smallpox vaccine, Yellow Fever vaccine, Adenovirus vaccine, Coxsackie B virus vaccine, Cytomegalovirus vaccine, Dengue vaccine for humans, Eastern Equine encephalitis virus vaccine for humans, Ebola vaccine, Enterovirus 71 vaccine, Epstein-Barr vaccine, Hepatitis C vaccine, HIV vaccine, HTLV-1 T-lymphotropic leukemia vaccine for humans, Marburg virus disease vaccine, Norovirus vaccine, Respiratory syncytial virus vaccine for humans, Severe acute respiratory syndrome (SARS) vaccine, West Nile virus vaccine for humans; Examples of bacterial diseases include but are not limited to: Anthrax vaccines, DPT vaccine, Q fever vaccine, Hib vaccine, Tuberculosis (BCG) vaccine, Meningococcal vaccine, Typhoid vaccine, Pneumococcal conjugate vaccine, Pneumococcal polysaccharide vaccine, Cholera vaccine, Caries vaccine, Ehrlichiosis vaccine, Leprosy vaccine, Lyme disease vaccine, Staphylococcus aureus vaccine, Streptococcus pyogenes vaccine, Syphilis vaccine, Tularemia vaccine, Yersinia pestis vaccine; Examples of parasitic diseases include but are not limited to: Malaria vaccine, Schistosomiasis vaccine, Chagas disease vaccine, Hookworm vaccine, Onchocerciasis river blindness vaccine for humans, Trypanosomiasis vaccine, Visceral leishmaniasis vaccine; Examples of non-infectious diseases include but are not limited to: Alzheimer's disease amyloid protein vaccine, Breast cancer vaccine, Ovarian cancer vaccine, Prostate cancer vaccine, Talimogene laherparepvec (T-VEC); also vaccines including but not limited to the following trade names: ACAM2000, ActHIB, Adacel, Afluria, AFLURIA QUADRIVALENT, Agriflu, BCG Vaccine, BEXSERO, Biothrax, Boostrix, Cervarix, Comvax, DAPTACEL, DECAVAC, Engerix-B, FLUAD, Fluarix, Fluarix Quadrivalent, Flublok, Flucelvax, Flucelvax Quadrivalent, FluLaval, FluMist, FluMist Quadrivalent, Fluvirin, Fluzone Quadrivalent, Fluzone, Fluzone High-Dose and Fluzone Intradermal, Gardasil, Gardasil 9, Havrix, Hiberix, Imovax, Infanrix, IPOL, Ixiaro, JE-Vax, KINRIX, Menactra, MenHibrix, Menomune-A/C/Y/W-135, Menveo, M-M-R II, M-M-Vax, Pediarix, PedvaxHIB, Pentacel, Pneumovax 23, Poliovax, Prevnar, Prevnar 13, ProQuad, Quadracel, Quadrivalent, RabAvert, Recombivax HB, ROTARIX, RotaTeq, TENIVAC, TICE BCG, Tripedia, TRUMENBA, Twinrix, TYPHIM Vi, VAQTA, Varivax, Vaxchora, Vivotif, YF-Vax, Zostavax, and combinations thereof.

Examples of injectable drugs include but are not limited to: Ablavar (Gadofosveset Trisodium Injection), Abarelix Depot, Abobotulinumtoxin A Injection (Dysport), ABT-263, ABT-869, ABX-EFG, Accretropin (Somatropin Injection), Acetadote (Acetylcysteine Injection), Acetazolamide Injection (Acetazolamide Injection), Acetylcysteine Injection (Acetadote), Actemra (Tocilizumab Injection), Acthrel (Corticorelin Ovine Triflutate for Injection), Actummune, Activase, Acyclovir for Injection (Zovirax Injection), [0137], Adacel, Adalimumab, Adenoscan (Adenosine Injection), Adenosine Injection (Adenoscan), Adrenaclick, AdreView (lobenguane 1123 Injection for Intravenous Use), Afluria, Ak-Fluor (Fluorescein Injection), Aldurazyme (Laronidase), Alglucerase Injection (Ceredase), Alkeran Injection (Melphalan Hcl Injection), Allopurinol Sodium for Injection (Aloprim), Aloprim (Allopurinol Sodium for Injection), Alprostadil, Alsuma (Sumatriptan Injection), ALTU-238, Amino Acid Injections, Aminosyn, Apidra, Apremilast, Alprostadil Dual Chamber System for Injection (Caverject Impulse), AMG 009, AMG 076, AMG 102, AMG 108, AMG 114, AMG 162, AMG 220, AMG 221, AMG 222, AMG 223, AMG 317, AMG 379, AMG 386, AMG 403, AMG 477, AMG 479, AMG 517, AMG 531, AMG 557, AMG 623, AMG 655, AMG 706, AMG 714, AMG 745, AMG 785, AMG 811, AMG 827, AMG 837, AMG 853, AMG 951, Amiodarone HCl Injection (Amiodarone HCl Injection), Amobarbital Sodium Injection (Amytal Sodium), Amytal Sodium (Amobarbital Sodium Injection), Anakinra, Anti-Abeta, Anti-Beta7, Anti-Beta20, Anti-CD4, Anti-CD20, Anti-CD40, Anti-IFNalpha, Anti-IL13, Anti-OX40L, Anti-oxLDS, Anti-NGF, Anti-NRP1, Arixtra, Amphadase (Hyaluronidase Inj), Ammonul (Sodium Phenylacetate and Sodium Benzoate Injection), Anaprox, Anzemet Injection (Dolasetron Mesylate Injection), Apidra (Insulin Glulisine [rDNA origin] Inj), Apomab, Aranesp (darbepoetin alfa), Argatroban (Argatroban Injection), Arginine Hydrochloride Injection (R-Gene 10, Aristocort, Aristospan, Arsenic Trioxide Injection (Trisenox), Articane HCl and Epinephrine Injection (Septocaine), Arzerra (Ofatumumab Injection), Asclera (Polidocanol Injection), Ataluren, Ataluren-DMD, Atenolol Inj (Tenormin I.V. Injection), Atracurium Besylate Injection (Atracurium Besylate Injection), Avastin, Azactam Injection (Aztreonam Injection), Azithromycin (Zithromax Injection), Aztreonam Injection (Azactam Injection), Baclofen Injection (Lioresal Intrathecal), Bacteriostatic Water (Bacteriostatic Water for Injection), Baclofen Injection (Lioresal Intrathecal), Bal in Oil Ampules (Dimercarprol Injection), BayHepB, BayTet, Benadryl, Bendamustine Hydrochloride Injection (Treanda), Benztropine Mesylate Injection (Cogentin), Betamethasone Injectable Suspension (Celestone Soluspan), Bexxar, Bicillin C-R 900/300 (Penicillin G Benzathine and Penicillin G Procaine Injection), Blenoxane (Bleomycin Sulfate Injection), Bleomycin Sulfate Injection (Blenoxane), Boniva Injection (Ibandronate Sodium Injection), Botox Cosmetic (OnabotulinumtoxinA for Injection), BR3-FC, Bravelle (Urofollitropin Injection), Bretylium (Bretylium Tosylate Injection), Brevital Sodium (Methohexital Sodium for Injection), Brethine, Briobacept, BTT-1023, Bupivacaine HCl, Byetta, Ca-DTPA (Pentetate Calcium Trisodium Inj), Cabazitaxel Injection (Jevtana), Caffeine Alkaloid (Caffeine and Sodium Benzoate Injection), Calcijex Injection (Calcitrol), Calcitrol (Calcijex Injection), Calcium Chloride (Calcium Chloride Injection 10%), Calcium Disodium Versenate (Edetate Calcium Disodium Injection), Campath (Altemtuzumab), Camptosar Injection (Irinotecan Hydrochloride), Canakinumab Injection (Ilaris), Capastat Sulfate (Capreomycin for Injection), Capreomycin for Injection (Capastat Sulfate), Cardiolite (Prep kit for Technetium Tc99 Sestamibi for Injection), Carticel, Cathflo, Cefazolin and Dextrose for Injection (Cefazolin Injection), Cefepime Hydrochloride, Cefotaxime, Ceftriaxone, Cerezyme, Carnitor Injection, Caverject, Celestone Soluspan, Celsior, Cerebyx (Fosphenytoin Sodium Injection), Ceredase (Alglucerase Injection), Ceretec (Technetium Tc99m Exametazime Injection), Certolizumab, CF-101, Chloramphenicol Sodium Succinate (Chloramphenicol Sodium Succinate Injection), Chloramphenicol Sodium Succinate Injection (Chloramphenicol Sodium Succinate), Cholestagel (Colesevelam HCL), Choriogonadotropin Alfa Injection (Ovidrel), Cimzia, Cisplatin (Cisplatin Injection), Clolar (Clofarabine Injection), Clomiphine Citrate, Clonidine Injection (Duraclon), Cogentin (Benztropine Mesylate Injection), Colistimethate Injection (Coly-Mycin M), Coly-Mycin M (Colistimethate Injection), Compath, Conivaptan Hcl Injection (Vaprisol), Conjugated Estrogens for Injection (Premarin Injection), Copaxone, Corticorelin Ovine Triflutate for Injection (Acthrel), Corvert (Ibutilide Fumarate Injection), Cubicin (Daptomycin Injection), CF-101, Cyanokit (Hydroxocobalamin for Injection), Cytarabine Liposome Injection (DepoCyt), Cyanocobalamin, Cytovene (ganciclovir), D.H.E. 45, Dacetuzumab, Dacogen (Decitabine Injection), Dalteparin, Dantrium IV (Dantrolene Sodium for Injection), Dantrolene Sodium for Injection (Dantrium IV), Daptomycin Injection (Cubicin), Darbepoietin Alfa, DDAVP Injection (Desmopressin Acetate Injection), Decavax, Decitabine Injection (Dacogen), Dehydrated Alcohol (Dehydrated Alcohol Injection), Denosumab Injection (Prolia), Delatestryl, Delestrogen, Delteparin Sodium, Depacon (Valproate Sodium Injection), Depo Medrol (Methylprednisolone Acetate Injectable Suspension), DepoCyt (Cytarabine Liposome Injection), DepoDur (Morphine Sulfate XR Liposome Injection), Desmopressin Acetate Injection (DDAVP Injection), Depo-Estradiol, Depo-Provera 104 mg/ml, Depo-Provera 150 mg/ml, Depo-Testosterone, Dexrazoxane for Injection, Intravenous Infusion Only (Totect), Dextrose/Electrolytes, Dextrose and Sodium Chloride Inj (Dextrose 5% in 0.9% Sodium Chloride), Dextrose, Diazepam Injection (Diazepam Injection), Digoxin Injection (Lanoxin Injection), Dilaudid-HP (Hydromorphone Hydrochloride Injection), Dimercarprol Injection (Bal in Oil Ampules), Diphenhydramine Injection (Benadryl Injection), Dipyridamole Injection (Dipyridamole Injection), DMOAD, Docetaxel for Injection (Taxotere), Dolasetron Mesylate Injection (Anzemet Injection), Doribax (Doripenem for Injection), Doripenem for Injection (Doribax), Doxercalciferol Injection (Hectorol Injection), Doxil (Doxorubicin Hcl Liposome Injection), Doxorubicin Hcl Liposome Injection (Doxil), Duraclon (Clonidine Injection), Duramorph (Morphine Injection), Dysport (Abobotulinumtoxin A Injection), Ecallantide Injection (Kalbitor), EC-Naprosyn (naproxen), Edetate Calcium Disodium Injection (Calcium Disodium Versenate), Edex (Alprostadil for Injection), Engerix, Edrophonium Injection (Enlon), Eliglustat Tartate, Eloxatin (Oxaliplatin Injection), Emend Injection (Fosaprepitant Dimeglumine Injection), Enalaprilat Injection (Enalaprilat Injection), Enlon (Edrophonium Injection), Enoxaparin Sodium Injection (Lovenox), Eovist (Gadoxetate Disodium Injection), Enbrel (etanercept), Enoxaparin, Epicel, Epinepherine, Epipen, Epipen Jr., Epratuzumab, Erbitux, Ertapenem Injection (Invanz), Erythropoieten, Essential Amino Acid Injection (Nephramine), Estradiol Cypionate, Estradiol Valerate, Etanercept, Exenatide Injection (Byetta), Evlotra, Fabrazyme (Adalsidase beta), Famotidine Injection, FDG (Fludeoxyglucose F 18 Injection), Feraheme (Ferumoxytol Injection), Feridex I.V. (Ferumoxides Injectable Solution), Fertinex, Ferumoxides Injectable Solution (Feridex I.V.), Ferumoxytol Injection (Feraheme), Flagyl Injection (Metronidazole Injection), Fluarix, Fludara (Fludarabine Phosphate), Fludeoxyglucose F 18 Injection (FDG), Fluorescein Injection (Ak-Fluor), Follistim AQ Cartridge (Follitropin Beta Injection), Follitropin Alfa Injection (Gonal-f RFF), Follitropin Beta Injection (Follistim AQ Cartridge), Folotyn (Pralatrexate Solution for Intravenous Injection), Fondaparinux, Forteo (Teriparatide (rDNA origin) Injection), Fostamatinib, Fosaprepitant Dimeglumine Injection (Emend Injection), Foscarnet Sodium Injection (Foscavir), Foscavir (Foscarnet Sodium Injection), Fosphenytoin Sodium Injection (Cerebyx), Fospropofol Disodium Injection (Lusedra), Fragmin, Fuzeon (enfuvirtide), GA101, Gadobenate Dimeglumine Injection (Multihance), Gadofosveset Trisodium Injection (Ablavar), Gadoteridol Injection Solution (ProHance), Gadoversetamide Injection (OptiMARK), Gadoxetate Disodium Injection (Eovist), Ganirelix (Ganirelix Acetate Injection), Gardasil, GC1008, GDFD, Gemtuzumab Ozogamicin for Injection (Mylotarg), Genotropin, Gentamicin Injection, GENZ-112638, Golimumab Injection (Simponi Injection), Gonal-f RFF (Follitropin Alfa Injection), Granisetron Hydrochloride (Kytril Injection), Gentamicin Sulfate, Glatiramer Acetate, Glucagen, Glucagon, HAE1, Haldol (Haloperidol Injection), Havrix, Hectorol Injection (Doxercalciferol Injection), Hedgehog Pathway Inhibitor, Heparin, Herceptin, hG-CSF, Humalog, Human Growth Hormone, Humatrope, HuMax, Humegon, Humira, Humulin, Ibandronate Sodium Injection (Boniva Injection), Ibuprofen Lysine Injection (NeoProfen), Ibutilide Fumarate Injection (Corvert), Idamycin PFS (Idarubicin Hydrochloride Injection), Idarubicin Hydrochloride Injection (Idamycin PFS), Ilaris (Canakinumab Injection), Imipenem and Cilastatin for Injection (Primaxin I.V.), Imitrex, Incobotulinumtoxin A for Injection (Xeomin), Increlex (Mecasermin [rDNA origin] Injection), Indocin IV (Indomethacin Inj), Indomethacin Inj (Indocin IV), Infanrix, Innohep, Insulin, Insulin Aspart [rDNA origin] Inj (NovoLog), Insulin Glargine [rDNA origin] Injection (Lantus), Insulin Glulisine [rDNA origin] Inj (Apidra), Interferon alfa-2b, Recombinant for Injection (Intron A), Intron A (Interferon alfa-2b, Recombinant for Injection), Invanz (Ertapenem Injection), Invega Sustenna (Paliperidone Palmitate Extended-Release Injectable Suspension), Invirase (saquinavir mesylate), lobenguane 1123 Injection for Intravenous Use (AdreView), lopromide Injection (Ultravist), loversol Injection (Optiray Injection), Iplex (Mecasermin Rinfabate [rDNA origin] Injection), Iprivask, Irinotecan Hydrochloride (Camptosar Injection), Iron Sucrose Injection (Venofer), Istodax (Romidepsin for Injection), Itraconazole Injection (Sporanox Injection), Jevtana (Cabazitaxel Injection), Jonexa, Kalbitor (Ecallantide Injection), KCL in D5NS (Potassium Chloride in 5% Dextrose and Sodium Chloride Injection), KCL in D5W, KCL in NS, Kenalog 10 Injection (Triamcinolone Acetonide Injectable Suspension), Kepivance (Palifermin), Keppra Injection (Levetiracetam), Keratinocyte, KFG, Kinase Inhibitor, Kineret (Anakinra), Kinlytic (Urokinase Injection), Kinrix, Klonopin (clonazepam), Kytril Injection (Granisetron Hydrochloride), lacosamide Tablet and Injection (Vimpat), Lactated Ringer's, Lanoxin Injection (Digoxin Injection), Lansoprazole for Injection (Prevacid I.V.), Lantus, Leucovorin Calcium (Leucovorin Calcium Injection), Lente (L), Leptin, Levemir, Leukine Sargramostim, Leuprolide Acetate, Levothyroxine, Levetiracetam (Keppra Injection), Lovenox, Levocarnitine Injection (Carnitor Injection), Lexiscan (Regadenoson Injection), Lioresal Intrathecal (Baclofen Injection), Liraglutide [rDNA] Injection (Victoza), Lovenox (Enoxaparin Sodium Injection), Lucentis (Ranibizumab Injection), Lumizyme, Lupron (Leuprolide Acetate Injection), Lusedra (Fospropofol Disodium Injection), Maci, Magnesium Sulfate (Magnesium Sulfate Injection), Mannitol Injection (Mannitol IV), Marcaine (Bupivacaine Hydrochloride and Epinephrine Injection), Maxipime (Cefepime Hydrochloride for Injection), MDP Multidose Kit of Technetium Injection (Technetium Tc99m Medronate Injection), Mecasermin [rDNA origin] Injection (Increlex), Mecasermin Rinfabate [rDNA origin] Injection (Iplex), Melphalan Hcl Injection (Alkeran Injection), Methotrexate, Menactra, Menopur (Menotropins Injection), Menotropins for Injection (Repronex), Methohexital Sodium for Injection (Brevital Sodium), Methyldopate Hydrochloride Injection, Solution (Methyldopate Hcl), Methylene Blue (Methylene Blue Injection), Methylprednisolone Acetate Injectable Suspension (Depo Medrol), MetMab, Metoclopramide Injection (Reglan Injection), Metrodin (Urofollitropin for Injection), Metronidazole Injection (Flagyl Injection), Miacalcin, Midazolam (Midazolam Injection), Mimpara (Cinacalet), Minocin Injection (Minocycline Inj), Minocycline Inj (Minocin Injection), Mipomersen, Mitoxantrone for Injection Concentrate (Novantrone), Morphine Injection (Duramorph), Morphine Sulfate XR Liposome Injection (DepoDur), Morrhuate Sodium (Morrhuate Sodium Injection), Motesanib, Mozobil (Plerixafor Injection), Multihance (Gadobenate Dimeglumine Injection), Multiple Electrolytes and Dextrose Injection, Multiple Electrolytes Injection, Mylotarg (Gemtuzumab Ozogamicin for Injection), Myozyme (Alglucosidase alfa), Nafcillin Injection (Nafcillin Sodium), Nafcillin Sodium (Nafcillin Injection), Naltrexone XR Inj (Vivitrol), Naprosyn (naproxen), NeoProfen (Ibuprofen Lysine Injection), Nandrol Decanoate, Neostigmine Methylsulfate (Neostigmine Methylsulfate Injection), NEO-GAA, NeoTect (Technetium Tc 99m Depreotide Injection), Nephramine (Essential Amino Acid Injection), Neulasta (pegfilgrastim), Neupogen (Filgrastim), Novolin, Novolog, NeoRecormon, Neutrexin (Trimetrexate Glucuronate Inj), NPH (N), Nexterone (Amiodarone HCl Injection), Norditropin (Somatropin Injection), Normal Saline (Sodium Chloride Injection), Novantrone (Mitoxantrone for Injection Concentrate), Novolin 70/30 Innolet (70% NPH, Human Insulin Isophane Suspension and 30% Regular, Human Insulin Injection), NovoLog (Insulin Aspart [rDNA origin] Inj), Nplate (romiplostim), Nutropin (Somatropin (rDNA origin) for Inj), Nutropin AQ, Nutropin Depot (Somatropin (rDNA origin) for Inj), Octreotide Acetate Injection (Sandostatin LAR), Ocrelizumab, Ofatumumab Injection (Arzerra), Olanzapine Extended Release Injectable Suspension (Zyprexa Relprevv), Omnitarg, Omnitrope (Somatropin [ rDNA origin] Injection), Ondansetron Hydrochloride Injection (Zofran Injection), OptiMARK (Gadoversetamide Injection), Optiray Injection (loversol Injection), Orencia, Osmitrol Injection in Aviva (Mannitol Injection in Aviva Plastic Vessel 250), Osmitrol Injection in Viaflex (Mannitol Injection in Viaflex Plastic Vessel 250), Osteoprotegrin, Ovidrel (Choriogonadotropin Alfa Injection), Oxacillin (Oxacillin for Injection), Oxaliplatin Injection (Eloxatin), Oxytocin Injection (Pitocin), Paliperidone Palmitate Extended-Release Injectable Suspension (Invega Sustenna), Pamidronate Disodium Injection (Pamidronate Disodium Injection), Panitumumab Injection for Intravenous Use (Vectibix), Papaverine Hydrochloride Injection (Papaverine Injection), Papaverine Injection (Papaverine Hydrochloride Injection), Parathyroid Hormone, Paricalcitol Injection Fliptop Vial (Zemplar Injection), PARP Inhibitor, Pediarix, PEGIntron, Peginterferon, Pegfilgrastim, Penicillin G Benzathine and Penicillin G Procaine, Pentetate Calcium Trisodium Inj (Ca-DTPA), Pentetate Zinc Trisodium Injection (Zn-DTPA), Pepcid Injection (Famotidine Injection), Pergonal, Pertuzumab, Phentolamine Mesylate (Phentolamine Mesylate for Injection), Physostigmine Salicylate (Physostigmine Salicylate (injection)), Physostigmine Salicylate (injection) (Physostigmine Salicylate), Piperacillin and Tazobactam Injection (Zosyn), Pitocin (Oxytocin Injection), Plasma-Lyte 148 (Multiple Electrolytes Inj), Plasma-Lyte 56 and Dextrose (Multiple Electrolytes and Dextrose Injection in Viaflex, Plastic Vessel 250), PlasmaLyte, Plerixafor Injection (Mozobil), Polidocanol Injection (Asclera), Potassium Chloride, Pralatrexate Solution for Intravenous Injection (Folotyn), Pramlintide Acetate Injection (Symlin), Premarin Injection (Conjugated Estrogens for Injection), Prep kit for Technetium Tc99 Sestamibi for Injection (Cardiolite), Prevacid I.V. (Lansoprazole for Injection), Primaxin I.V. (Imipenem and Cilastatin for Injection), Prochymal, Procrit, Progesterone, ProHance (Gadoteridol Injection Solution), Prolia (Denosumab Injection), Promethazine HCl Injection (Promethazine Hydrochloride Injection), Propranolol Hydrochloride Injection (Propranolol Hydrochloride Injection), Quinidine Gluconate Injection (Quinidine Injection), Quinidine Injection (Quinidine Gluconate Injection), R-Gene 10 (Arginine Hydrochloride Injection), Ranibizumab Injection (Lucentis), Ranitidine Hydrochloride Injection (Zantac Injection), Raptiva, Reclast (Zoledronic Acid Injection), Recombivarix HB, Regadenoson Injection (Lexiscan), Reglan Injection (Metoclopramide Injection), Remicade, Renagel, Renvela (Sevelamer Carbonate), Repronex (Menotropins for Injection), Retrovir IV (Zidovudine Injection), rhApo2L/TRAIL, Ringer's and 5% Dextrose Injection (Ringers in Dextrose), Ringer's Injection (Ringers Injection), Rituxan, Rituximab, Rocephin (ceftriaxone), Rocuronium Bromide Injection (Zemuron), Roferon-A (interferon alfa-2a), Romazicon (flumazenil), Romidepsin for Injection (Istodax), Saizen (Somatropin Injection), Sandostatin LAR (Octreotide Acetate Injection), Sclerostin Ab, Sensipar (cinacalcet), Sensorcaine (Bupivacaine HCl Injections), Septocaine (Articane HCl and Epinephrine Injection), Serostim LQ (Somatropin (rDNA origin) Injection), Simponi Injection (Golimumab Injection), Sodium Acetate (Sodium Acetate Injection), Sodium Bicarbonate (Sodium Bicarbonate 5% Injection), Sodium Lactate (Sodium Lactate Injection in AVIVA), Sodium Phenylacetate and Sodium Benzoate Injection (Ammonul), Somatropin (rDNA origin) for Inj (Nutropin), Sporanox Injection (Itraconazole Injection), Stelara Injection (Ustekinumab), Stemgen, Sufenta (Sufentanil Citrate Injection), Sufentanil Citrate Injection (Sufenta), Sumavel, Sumatriptan Injection (Alsuma), Symlin, Symlin Pen, Systemic Hedgehog Antagonist, Synvisc-One (Hylan G-F 20 Single Intra-articular Injection), Tarceva, Taxotere (Docetaxel for Injection), Technetium Tc 99m, Telavancin for Injection (Vibativ), Temsirolimus Injection (Torisel), Tenormin I.V. Injection (Atenolol Inj), Teriparatide (rDNA origin) Injection (Forteo), Testosterone Cypionate, Testosterone Enanthate, Testosterone Propionate, Tev-Tropin (Somatropin, rDNA Origin, for Injection), tgAAC94, Thallous Chloride, Theophylline, Thiotepa (Thiotepa Injection), Thymoglobulin (Anti-Thymocyte Globulin (Rabbit), Thyrogen (Thyrotropin Alfa for Injection), Ticarcillin Disodium and Clavulanate Potassium Galaxy (Timentin Injection), Tigan Injection (Trimethobenzamide Hydrochloride Injectable), Timentin Injection (Ticarcillin Disodium and Clavulanate Potassium Galaxy), TNKase, Tobramycin Injection (Tobramycin Injection), Tocilizumab Injection (Actemra), Torisel (Temsirolimus Injection), Totect (Dexrazoxane for Injection, Intravenous Infusion Only), Trastuzumab-DM1, Travasol (Amino Acids (Injection)), Treanda (Bendamustine Hydrochloride Injection), Trelstar (Triptorelin Pamoate for Injectable Suspension), Triamcinolone Acetonide, Triamcinolone Diacetate, Triamcinolone Hexacetonide Injectable Suspension (Aristospan Injection 20 mg), Triesence (Triamcinolone Acetonide Injectable Suspension), Trimethobenzamide Hydrochloride Injectable (Tigan Injection), Trimetrexate Glucuronate Inj (Neutrexin), Triptorelin Pamoate for Injectable Suspension (Trelstar), Twinject, Trivaris (Triamcinolone Acetonide Injectable Suspension), Trisenox (Arsenic Trioxide Injection), Twinrix, Typhoid Vi, Ultravist (lopromide Injection), Urofollitropin for Injection (Metrodin), Urokinase Injection (Kinlytic), Ustekinumab (Stelara Injection), Ultralente (U), Valium (diazepam), Valproate Sodium Injection (Depacon), Valtropin (Somatropin Injection), Vancomycin Hydrochloride (Vancomycin Hydrochloride Injection), Vancomycin Hydrochloride Injection (Vancomycin Hydrochloride), Vaprisol (Conivaptan Hcl Injection), VAQTA, Vasovist (Gadofosveset Trisodium Injection for Intravenous Use), Vectibix (Panitumumab Injection for Intravenous Use), Venofer (Iron Sucrose Injection), Verteporfin Inj (Visudyne), Vibativ (Telavancin for Injection), Victoza (Liraglutide [rDNA] Injection), Vimpat (lacosamide Tablet and Injection), Vinblastine Sulfate (Vinblastine Sulfate Injection), Vincasar PFS (Vincristine Sulfate Injection), Victoza, Vincristine Sulfate (Vincristine Sulfate Injection), Visudyne (Verteporfin Inj), Vitamin B-12, Vivitrol (Naltrexone XR Inj), Voluven (Hydroxyethyl Starch in Sodium Chloride Injection), Xeloda, Xenical (orlistat), Xeomin (Incobotulinumtoxin A for Injection), Xolair, Zantac Injection (Ranitidine Hydrochloride Injection), Zemplar Injection (Paricalcitol Injection Fliptop Vial), Zemuron (Rocuronium Bromide Injection), Zenapax (daclizumab), Zevalin, Zidovudine Injection (Retrovir IV), Zithromax Injection (Azithromycin), Zn-DTPA (Pentetate Zinc Trisodium Injection), Zofran Injection (Ondansetron Hydrochloride Injection), Zingo, Zoledronic Acid for Inj (Zometa), Zoledronic Acid Injection (Reclast), Zometa (Zoledronic Acid for Inj), Zosyn (Piperacillin and Tazobactam Injection), Zyprexa Relprevv (Olanzapine Extended Release Injectable Suspension) and a combination thereof.

While the invention has been described in detail, modifications within the scope of the invention, as defined in the appended claims, will be readily apparent to the skilled artisan.

## Claims

1. A medical delivery device (10) comprising:
a lubricant-free barrel (15) having a proximal end (25), a distal end (20), and an interior surface defining a fluid chamber (30) for containment of at least one therapeutic;
an elastomeric stopper (100) having an outer surface forming a fluid-tight seal with the interior surface of the barrel (15), wherein the elastomeric stopper (100) comprises a plurality of ribs (120), each pair of ribs having a valley (115) therebetween;
**characterised in that** the medical delivery device further comprises
at least one ring member positioned within at least one valley (115),
wherein the at least one ring member comprises an oxygen-absorbing material, an oxygen-adsorbing material, or a combination thereof;
wherein the medical delivery device further comprises a coating material positioned on a portion of the interior surface of the barrel (15) adjacent to the proximal end (25) of the barrel (15) to form a region of reduced diameter to enhance an interference fit between the elastomeric stopper (100) and the interior surface of the barrel (15).

2. The medical delivery device (10) of claim 1, wherein two or more valleys (115) comprise said at least one ring member.

3. The medical delivery device (10) of any one of claims 1-2, wherein said at least one therapeutic is selected from DNA fragments of Target genes or DNA Target receptors genes of a group consisting of genes Coagulation Factors, Cytokines, Epigenetic protein families, Growth Factors, Hormones, Peptides, Signal Transduction molecules, Vaccines, and combinations thereof.

4. The medical delivery device (10) of any one of claims 1-3, wherein said therapeutic is selected from mutations of DNA fragments of Target genes or DNA Target receptors genes.

5. The medical delivery device (10) of any one of claims 1-4, wherein the therapeutic is factor VII.

6. The medical delivery device (10) of any one of claims 1-5, suitable for treatment of ocular disease.

7. The medical delivery device (10) of any one of claims 1-6, wherein the medical delivery device is a syringe, an auto-injector, or a pen.

8. A medical delivery device (10) comprising:
a silicone-free barrel having a proximal end, a distal end, and an interior surface defining a fluid chamber for containment of at least one therapeutic;
an elastomeric stopper having an outer surface forming a fluid-tight seal with the interior surface;
a plunger rod attached to the stopper and mounted within the barrel for moving the stopper distally within the barrel;
**characterised in that** the medical delivery device further comprises
a barrier coating positioned on at least a portion of the elastomeric stopper,
wherein the barrier coating comprises an oxygen barrier material; wherein a coating material is positioned on a portion of the interior surface of the barrel adjacent to the proximal end of the barrel to form a region of reduced diameter to enhance an interference fit between the elastomeric stopper and the interior surface of the barrel.

9. The medical delivery device (10) of claim 8, wherein the barrier coating surrounds a portion of the elastomeric stopper that is not exposed to the fluid chamber.

10. The medical delivery device (10) of claim 8 or claim 9, wherein the barrier coating surrounds the entirety of the elastomeric stopper.

11. The medical delivery device (10) of any one of claims 8-10, wherein the barrier coating is also positioned on the plunger rod.

12. The medical delivery device (10) of any one of claims 8-10, wherein the elastomeric stopper is at least partially covered with an expanded polytetrafluoroethylene layer or a densified expanded polytetrafluoroethylene layer.

13. The medical delivery device (10) of any one of claims 8-12, wherein said at least one therapeutic is selected from DNA fragments of Target genes or DNA Target receptors genes of a group consisting of genes Coagulation Factors, Cytokines, Epigenetic protein families, Growth Factors, Hormones, Peptides, Signal Transduction molecules, Vaccines, and combinations thereof.

14. The medical delivery device (10) of any one of claims 8-13, wherein said therapeutic is selected from mutations of DNA fragments of Target genes or DNA Target receptors genes.

15. The medical delivery device (10) of any one of claims 8-14, wherein the therapeutic is factor VII.

16. The medical delivery device (10) of any one of claims 8-15, suitable for treatment
of ocular disease.

17. The medical delivery device (10) of any one of claims 8-16, wherein the medical delivery device is a syringe, an auto-injector, or a pen.

## Patentansprüche

1. Medizinische Freisetzungsvorrichtung (10), umfassend:
einen schmiermittelfreien Zylinder (15) mit einem proximalen Ende (25), einem distalen Ende (20) und einer Innenfläche, wodurch eine Flüssigkeitskammer (30) zur Rückhaltung mindestens eines Therapeutikums definiert wird;
einen elastomeren Stopfen (100) mit einer Außenfläche, der eine flüssigkeitsdichte Abdichtung mit der Innenfläche des Zylinders (15) bildet, wobei der elastomere Stopfen (100) eine Vielzahl von Rippen (120) umfasst, wobei jedes Rippenpaar ein Tal (115) dazwischen aufweist;
**dadurch gekennzeichnet, dass** die medizinische Freisetzungsvorrichtung ferner mindestens ein Ringelement umfasst, das innerhalb mindestens eines Tals (115) positioniert ist,
wobei das mindestens eine Ringelement ein sauerstoffabsorbierendes Material, ein sauerstoffadsorbierendes Material oder eine Kombination davon umfasst;
wobei die medizinische Freisetzungsvorrichtung ferner ein Beschichtungsmaterial umfasst, das auf einem Abschnitt der Innenfläche des Zylinders (15) neben dem proximalen Ende (25) des Zylinders (15) positioniert ist, um einen Bereich mit verringertem Durchmesser zu bilden, um eine Presspassung zwischen dem elastomeren Stopfen (100) und der Innenfläche des Zylinders (15) zu verbessern.

2. Medizinische Freisetzungsvorrichtung (10) nach Anspruch 1, wobei zwei oder mehr Täler (115) das mindestens eine Ringelement umfassen.

3. Medizinische Freisetzungsvorrichtung (10) nach einem der Ansprüche 1-2, wobei das mindestens eine Therapeutikum aus DNA-Fragmenten von Zielgenen oder DNA-Zielrezeptorgenen einer Gruppe ausgewählt ist, die aus Genen Gerinnungsfaktoren, Zytokinen, epigenetischen Proteinfamilien, Wachstumsfaktoren, Hormonen, Peptiden, Signaltransduktionsmolekülen, Impfstoffen und Kombinationen davon besteht.

4. Medizinische Freisetzungsvorrichtung (10) nach einem der Ansprüche 1-3, wobei das Therapeutikum aus Mutationen von DNA-Fragmenten von Zielgenen oder DNA-Zielrezeptorgenen ausgewählt ist.

5. Medizinische Freisetzungsvorrichtung (10) nach einem der Ansprüche 1-4, wobei das Therapeutikum Faktor VII ist.

6. Medizinische Freisetzungsvorrichtung (10) nach einem der Ansprüche 1-5, die für die Behandlung von Augenkrankheiten geeignet ist.

7. Medizinische Freisetzungsvorrichtung (10) nach einem der Ansprüche 1-6, wobei die medizinische Freisetzungsvorrichtung eine Spritze, ein Autoinjektor oder ein Pen ist.

8. Medizinische Freisetzungsvorrichtung (10), umfassend:
einen silikonfreien Zylinder mit einem proximalen Ende, einem distalen Ende und einer Innenfläche, wodurch eine Flüssigkeitskammer zur Rückhaltung mindestens eines Therapeutikums definiert wird;
einen elastomeren Stopfen mit einer Außenfläche, der eine flüssigkeitsdichte Abdichtung mit der Innenfläche bildet;
eine Kolbenstange, die an dem Stopfen angebracht und innerhalb des Zylinders montiert ist, um den Stopfen distal innerhalb des Zylinders zu bewegen;
**dadurch gekennzeichnet, dass** die medizinische Freisetzungsvorrichtung ferner eine Barrierebeschichtung umfasst, die auf mindestens einem Abschnitt des elastomeren Stopfens positioniert ist, wobei die Barrierebeschichtung ein Sauerstoffbarrierematerial umfasst; wobei ein Beschichtungsmaterial auf einem Abschnitt der Innenfläche des Zylinders neben dem proximalen Ende des Zylinders positioniert ist, um einen Bereich mit verringertem Durchmesser zu bilden,
um eine Presspassung zwischen dem elastomeren Stopfen und der Innenfläche des Zylinders zu verbessern.

9. Medizinische Freisetzungsvorrichtung (10) nach Anspruch 8, wobei die Barrierebeschichtung einen Abschnitt des elastomeren Stopfens umgibt, der nicht der Flüssigkeitskammer ausgesetzt ist.

10. Medizinische Freisetzungsvorrichtung (10) nach Anspruch 8 oder Anspruch 9, wobei die Barrierebeschichtung die Gesamtheit des elastomeren Stopfens umgibt.

11. Medizinische Freisetzungsvorrichtung (10) nach einem der Ansprüche 8-10, wobei die Barrierebeschichtung ebenfalls auf der Kolbenstange positioniert ist.

12. Medizinische Freisetzungsvorrichtung (10) nach einem der Ansprüche 8-10, wobei der elastomere Stopfen mindestens teilweise mit einer expandierten Polytetrafluorethylenschicht oder einer verdichteten expandierten Polytetrafluorethylenschicht bedeckt ist.

13. Medizinische Freisetzungsvorrichtung (10) nach einem der Ansprüche 8-12, wobei das mindestens eine Therapeutikum aus DNA-Fragmenten von Zielgenen oder DNA-Zielrezeptorgenen einer Gruppe ausgewählt ist, die aus Genen Gerinnungsfaktoren, Zytokinen, epigenetischen Proteinfamilien, Wachstumsfaktoren, Hormonen, Peptiden, Signaltransduktionsmolekülen, Impfstoffen und Kombinationen davon besteht.

14. Medizinische Freisetzungsvorrichtung (10) nach einem der Ansprüche 8-13, wobei das Therapeutikum aus Mutationen von DNA-Fragmenten von Zielgenen oder DNA-Zielrezeptorgenen ausgewählt ist.

15. Medizinische Freisetzungsvorrichtung (10) nach einem der Ansprüche 8-14, wobei das Therapeutikum Faktor VII ist.

16. Medizinische Freisetzungsvorrichtung (10) nach einem der Ansprüche 8-15, die für die Behandlung von Augenkrankheiten geeignet ist.

17. Medizinische Freisetzungsvorrichtung (10) nach einem der Ansprüche 8-16, wobei die medizinische Freisetzungsvorrichtung eine Spritze, ein Autoinjektor oder ein Pen ist.

## Revendications

1. Dispositif d'administration médicale (10) comprenant :
un cylindre exempt de lubrifiant (15) comportant une extrémité proximale (25), une extrémité distale (20) et une surface interne définissant une chambre à fluide (30) destinée à contenir au moins un agent thérapeutique ;
un bouchon élastomère (100) comportant une surface externe formant un joint étanche aux fluides avec la surface interne du cylindre (15), ledit bouchon élastomère (100) comprenant une pluralité de nervures (120), chaque paire de nervures comportant un creux (115) entre elles ;
**caractérisé en ce que** le dispositif d'administration médicale comprend en outre au moins un élément annulaire positionné à l'intérieur d'au moins un creux (115),
ledit au moins un élément annulaire comprenant un matériau absorbant l'oxygène, un matériau adsorbant l'oxygène ou une combinaison de ceux-ci ;
ledit dispositif d'administration médicale comprenant en outre un matériau de revêtement positionné sur une partie de la surface interne du cylindre (15) adjacente à l'extrémité proximale (25) du cylindre (15) de manière à former une zone de diamètre réduit permettant d'améliorer un ajustement serré entre le bouchon élastomère (100) et la surface interne du cylindre (15).

2. Dispositif d'administration médicale (10) selon la revendication 1, deux creux (115) ou plus comprenant ledit au moins un élément annulaire.

3. Dispositif d'administration médicale (10) selon l'une quelconque des revendications 1 à 2, ledit au moins un agent thérapeutique étant choisi parmi des fragments d'ADN de gènes cibles ou de gènes de récepteurs cibles d'ADN d'un groupe constitué par les gènes des facteurs de coagulation, des cytokines, des familles des protéines épigénétiques, des facteurs de croissance, des hormones, des peptides, des molécules de transduction de signal, des vaccins et leurs combinaisons.

4. Dispositif d'administration médicale (10) selon l'une quelconque des revendications 1 à 3, ledit agent thérapeutique étant choisi parmi des mutations de fragments d'ADN de gènes cibles ou de gènes de récepteurs cibles d'ADN.

5. Dispositif d'administration médicale (10) selon l'une quelconque des revendications 1 à 4, ledit agent thérapeutique étant le facteur VII.

6. Dispositif d'administration médicale (10) selon l'une quelconque des revendications 1 à 5, adapté au traitement d'une maladie oculaire.

7. Dispositif d'administration médicale (10) selon l'une quelconque des revendications 1 à 6, ledit dispositif d'administration médicale étant une seringue, un auto-injecteur ou un stylo.

8. Dispositif d'administration médicale (10) comprenant :
un cylindre exempt de silicone comportant une extrémité proximale, une extrémité distale et une surface interne définissant une chambre à fluide destinée à contenir au moins un agent thérapeutique ;
un bouchon élastomère comportant une surface externe formant un joint étanche aux fluides avec la surface interne ;
une tige de piston fixée au bouchon et montée à l'intérieur du cylindre destinée à déplacer le bouchon de manière distale à l'intérieur du cylindre ;
**caractérisé en ce que** le dispositif d'administration médicale comprend en outre un revêtement barrière positionné sur au moins une partie du bouchon élastomère,
ledit revêtement barrière comprenant un matériau faisant barrière à l'oxygène ; un matériau de revêtement étant positionné sur une partie de la surface interne du cylindre adjacente à l'extrémité proximale du cylindre de manière à former une zone de diamètre réduit permettant d'améliorer un ajustement serré entre le bouchon élastomère et la surface interne du cylindre.

9. Dispositif d'administration médicale (10) selon la revendication 8, ledit revêtement barrière entourant une partie du bouchon élastomère qui n'est pas exposée à la chambre à fluide.

10. Dispositif d'administration médicale (10) selon la revendication 8 ou la revendication 9, ledit revêtement barrière entourant la totalité du bouchon élastomère.

11. Dispositif d'administration médicale (10) selon l'une quelconque des revendications 8 à 10, ledit revêtement barrière étant également positionné sur la tige de piston.

12. Dispositif d'administration médicale (10) selon l'une quelconque des revendications 8 à 10, ledit bouchon élastomère étant au moins partiellement recouvert d'une couche de polytétrafluoroéthylène expansé ou d'une couche de polytétrafluoroéthylène expansé densifié.

13. Dispositif d'administration médicale (10) selon l'une quelconque des revendications 8 à 12, ledit au moins un agent thérapeutique étant choisi parmi des fragments d'ADN de gènes cibles ou de gènes de récepteurs cibles d'ADN d'un groupe constitué par les gènes des facteurs de coagulation, des cytokines, des familles des protéines épigénétiques, des facteurs de croissance, des hormones, des peptides, des molécules de transduction de signal, des vaccins et leurs combinaisons.

14. Dispositif d'administration médicale (10) selon l'une quelconque des revendications 8 à 13, ledit agent thérapeutique étant choisi parmi des mutations de fragments d'ADN de gènes cibles ou de gènes de récepteurs cibles d'ADN.

15. Dispositif d'administration médicale (10) selon l'une quelconque des revendications 8 à 14, ledit agent thérapeutique étant le facteur VII.

16. Dispositif d'administration médicale (10) selon l'une quelconque des revendications 8 à 15, adapté au traitement d'une maladie oculaire.

17. Dispositif d'administration médicale (10) selon l'une quelconque des revendications 8 à 16, ledit dispositif d'administration médicale étant une seringue, un auto-injecteur ou un stylo.
